(19) Europäisches Patentamt
European Patent Office
Office européen des brevets

(11) EP 4 613 730 A1

(12) EUROPEAN PATENT APPLICATION

(43) Date of publication:
**10.09.2025 Bulletin 2025/37**

(21) Application number: **24305335.2**

(22) Date of filing: **05.03.2024**

(51) International Patent Classification (IPC):
***C07C 33/044*** *(2006.01)* ***C07C 33/048*** *(2006.01)*
***C07C 33/05*** *(2006.01)* ***C07C 33/12*** *(2006.01)*
***C07C 33/14*** *(2006.01)* ***C07C 33/30*** *(2006.01)*

(52) Cooperative Patent Classification (CPC):
**C07C 33/30; C07C 33/044; C07C 33/048; C07C 33/05; C07C 33/12; C07C 33/14;**
C07C 2601/02; C07C 2601/08; C07C 2601/14

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **Centre National de la Recherche Scientifique 75016 Paris (FR)**
- **UNIVERSITE TOULOUSE III - PAUL SABATIER 31062 Toulouse Cedex 9 (FR)**

(72) Inventor: **The designation of the inventor has not yet been filed**

(74) Representative: **Lavoix**
**2, place d'Estienne d'Orves**
**75441 Paris Cedex 09 (FR)**

# (54) LIPIDIC ALKYNYLCARBINOLS WITH ANTI-BACTERIAL AND ANTI-TUBERCULOSIS ACTIVITY

(57) The present invention relates to novel synthetic lipidic alkynylcarbinols, in particular as antibacterial agents, preferably against mycobacteria, and more preferably against species from the *Mycobacterium tuberculosis* complex, and also to therapeutic uses thereof. The present invention also relates to said compounds for use for the treatment of bacterial infections.

The present invention further relates to the discovery of the pharmaceutical mechanism and bioactivity of these novel synthetic lipidic alkynylcarbinols.

EP 4 613 730 A1

## Description

The present invention relates to the field of bacterial or parasitic infections and in particular tuberculosis.

A special attention was focused on the mycobacteria, *Mycobacterium tuberculosis* (*Mtb*). Indeed tuberculosis remains one of the most deadly diseases in the world, despite the availability of a vaccine (BCG, "Bacille Calmette-Guérin" invented more than a century ago) and a long-proven antibiotic therapy (DOTS strategy, "directly observed treatment, short-course"). The DOTS protocol, implemented for more than 50 years, is based on the administration of the 4 first-line antibiotics, isoniazid (1952), pyrazinamide (1954), ethambutol (1961) and rifampicin (1963), while second-line drugs against resistant strains, such as fluoroquinolones, long dated back to the 1980s. More recent approvals of bedaquiline, delamanid or pretomanid may allow new hopes (Stover et al., Nature. June 2000, Vol. 405, pages 962-966; Matsumoto et al., PLoS Med. November, 2006, Vol.3, No.11, e466; Andries et al., Science. January 2005, Vol.307, Issue 5707, Pages 223-227) but the explosive emergence of multi-resistant strains of *Mtb* made urgent the development of molecules of new structural types. Alternative molecules from various natural families have thus been reported (mainly heterocycles, alkaloids or peptides, and some non-nitrogenous phenols, quinones, terpenoids or steroids), but their activity remained relatively low (IC50 > 10 μM). (for representative early examples : Palomino et al., Curr. Med. Chem. May 2009, Vol.16, No.15, Pages 1898-1904; Rogoza et al., Chem. Sustain. Development 2010, Vol.10, Pages 343-375).

A few natural acetylenic lipids, such as exocarpic acid extracted from Exocarpos latifolius were thus described to be weak anti-*Mtb* agents (Koch et al., Planta Med. 2010. Vol.75, No.15, Pages 1678-1682), while lipidic alkynoic acids, which happen to be simplified versions of mycomycin, were shown to be active against *Mycobacterium smegmatis* and *Mycobacterium bovis* (Morbidoni et al., Chemistry & Biology. March 2006. Vol.13, Issue 3, Pages 297-307). In the lipidic alkynylcarbinols family, natural lipidic alkynylcarbinols extracted from carrot roots, such as falcarinol and falcarindiol, have also been reported to be moderately active (Li et al., J. Ethnopharmacol. March 2012. Vol.140, Issue 1, Pages 141-144).

A novel subclass of particular linear lipidic derivatives was recently designed, namely the so-called lipidic alkynylcarbinols for sp-C-alkylated secondary propargylic alcohols (E! Arfaoui et al., ChemMedChem. September 2013. Vol.8, Pages 1779-1786; Listunov et al., Synthesis. 2018. Vol.50, No.16, Pages 3114-3130; Demange et al., eLife. May 2022. Vol. 1, e73913) and, more recently, aromatic derivatives thereof (Bossuat et al., J. Med. Chem. October 2023. Vol.66, Issue.20, Pages 13918-13945). Nevertheless, they are only concerned with anti-cancer activity.

Thus, there is an urgent need for new treatments against tuberculosis. Ideally, a new antituberculosis drug should exhibit antibacterial activity at low concentration, with a minimum inhibitory concentration (MIC) in the micromolar (μM) range or lower. Moreover, it should have a good selectivity with respect to mycobacteria, with low cytotoxicity towards mammalian cells, which can be estimated by the selectivity index (SI).

In 2006, a publication by Morbidoni et al., entitled "Dual inhibition of mycobacterial fatty acid biosynthesis and degradation by 2-alkynoic acids", Chemistry & Biology 2006, 13, 3, 297-307, reported the discovery of 2-alkynoic acids comprising an alkynyl group bonded to a carboxylic acid group on one side and a lipophilic tail on the other, which exhibit anti-bacterial effect against mycobacteria. In particular, they found 2-hexadecynoic acid and 2-octadecynoic acid to be the most effective compounds against *M. smegmatis* and *M. bovis* BCG.

However, the 2-alkynoic acids discovered by Morbioni *et al.* comprise a carboxylic acid group at the end of molecule chains. The carboxylic acid group makes these compounds amphiphilic, while consequently, these compounds also exhibit properties as surfactants, which have the potential of disrupting cellular membranes and eventually leading to nonspecific cell death, i.e. without discrimination between human cells and those of microorganisms. In addition, the therapeutic application of these 2-alkynoic acids against *Mtb* was not mentioned or studied.

Few publications have disclosed the therapeutic application of the alkynylcarbinol-containing compounds for the treatment of infections caused by *Mtb* and their anti-*Mtb* mechanism is yet not clear. These studies essentially relate to natural compounds derived from traditional herbal medicines, such as falcarindiol reported by Deng et al. entitled "Anti-TB Polyynes from Roots of Angelica sinensis", Phytotherapy Research 2008, 22, 878-882.

One of the objectives of the present invention is to provide novel synthetic lipidic alkynylcarbinols exhibiting a significant anti-bacterial activity, in particular a high activity against mycobacteria, and preferably a high activity against species from the *Mtb* complex. In addition, these novel synthetic lipidic alkynylcarbinols exhibit a larger structural variability with respect to the reported 2-alkynoic acids or natural compounds, which allows the optimization of the pharmacological performance by reasonable design of molecule structures.

The present invention further relates to the discovery of the pharmaceutical mechanism and bioactivity of these novel lipidic alkynylcarbinols.

Thus, the present invention relates to compounds having the following formula (I)

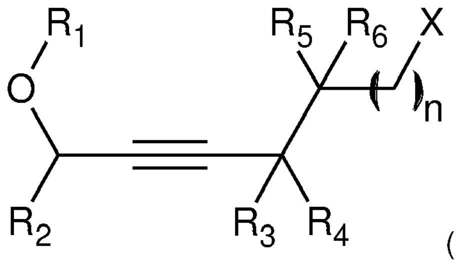

(I),

wherein:

- n is an integer comprised between 4 and 15;
- $R_1$ is Hydrogen, or a -C(=O)-R' or an alcohol protecting group;
- $R_2$ is chosen from the group consisting of: Hydrogen, Alkyl, Cycloalkyl, Heterocycle, Alkenyl, Allenyl, Alkynyl, Aryl, and Heteroaryl;
- $R_3$, $R_4$, $R_5$ and $R_6$ identical or different are independently chosen from the group consisting of Hydrogen, Halogen, Alkyl, Alkenyl, Alkynyl, AlkylAryl, $OR_a$, $COOR_b$, $NR_cR_d$, wherein at least one of $R_3$, $R_4$, $R_5$ and $R_6$ is different from H;
- Alternatively, groups $R_3$ and $R_4$ or groups $R_5$ and $R_6$ can form double carbon-oxygen bond (C=O);
- X is selected from the group consisting of $CH_3$, $CH_2F$, $CHF_2$, $CHFCH_2F$, $CF_3$, COOH, $CH_2OH$, alkoxy, $CCSi(i\text{-}Pr)_3$, alkynyl, $NH_2$, $-O-P(=O)(O_2)M$ and $-O-S(=O)_2-OM$;

wherein:

- M is one or more mono- or divalent metal ions chosen from Li+, $Na^+$, $Mg^{2+}$, $K^+$ and $Ca^{2+}$;
- $R_a$, $R_b$, $R_c$, $R_d$, identical or different, are independently chosen from H, Alkyl and - C(=O)-R' groups;
- R' is chosen from Hydrogen, Alkyl, Halogen, $OR_e$, $NR_fR_g$ , Cycloalkyl, and Aryl, wherein $R_e$, $R_f$, $R_g$ identical or different, are independently chosen from H and Alkyl;

wherein each of Alkyl, Alkenyl, Allenyl, Alkynyl, Cycloalkyl, Heterocycle, Aryl, Heteroaryl may be optionally substituted by one or more group chosen from the group consisting of: halogen, $OR_a$, $COOR_b$, $NR_cR_d$, CN, $NO_2$, $CF_3$;

as well as the stereoisomers thereof,
or a pharmaceutically acceptable salt or ester thereof.

As used herein:

- Alkyl refers to a linear or branched alkyl group;
- Alcohol protecting group refers to Aryl, Heterocycles, alkyl ether, silyl, or glycosyl;
- Cycloalkyl refers to a mono, bi or tricyclic carbocyclic ring;
- Heterocycle refers to a mono, bi or tricyclic saturated or unsaturated ring comprising one or more Heteroatoms chosen from N, O and S;
- Alkenyl refers to a linear or branched alkenyl group comprising one or more double bonds;
- Allenyl group refers to a -C(Ra)=C=CRbRc group;
- Alkynyl refers to a linear or branched alkynyl, comprising 1 or more triple bonds;
- Aryl refers to a mono, or bicyclic aromatic ring;
- Heteroaryl refers to a mono or bicyclic aromatic ring comprising one or more Heteroatoms chosen from N, O and S; and/or
- Halogen is chosen from F, Cl, Br and I.

In another object, the present invention also relates to compounds having the following formula (I')

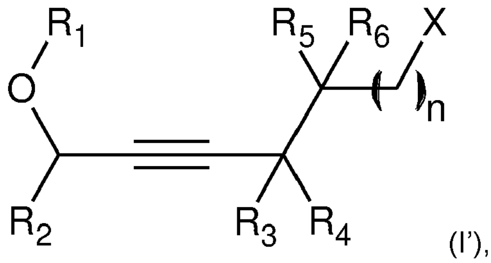

(I'),

wherein:

- n is an integer comprised between 4 and 15;
- $R_1$ is Hydrogen, or a -C(=O)-R' or an alcohol protecting group;
- $R_2$ is an alkynyl substituted by Alkyl-OH group;
- $R_3$, $R_4$, $R_5$ and $R_6$ identical or different are independently chosen from the group consisting of Hydrogen, Halogen, Alkyl, Alkenyl, Alkynyl, AlkylAryl, $OR_a$, $COOR_b$, $NR_cR_d$;
- Alternatively, groups $R_3$ and $R_4$ or groups $R_5$ and $R_6$ can form double carbon-oxygen bond (C=O);
- X is selected from the group consisting of $CH_3$, $CH_2F$, $CHF_2$, $CHFCH_2F$, $CF_3$, COOH, $CH_2OH$, alkoxy, $CCSi(i\text{-}Pr)_3$, alkynyl, $NH_2$, $-O-P(=O)(O_2)M$ and $-O-S(=O)_2-OM$;

wherein M, $R_a$, $R_b$, $R_c$, $R_d$ and R' are defined in the same way as formula (I);

as well as the stereoisomers thereof,
or a pharmaceutically acceptable salt or ester thereof.

According to an embodiment in formula (I'), $R_2$ is $-C{\equiv}C-CH_2OH$.

The following definitions are set forth merely to illustrate the various terms used to describe the invention herein.

The term "n" is an integer referring to the number of $-CH_2-$ groups, ranging from 1 to 20, preferably from 4 to 15, more preferably from 8 to 12.

The expression "Ct-Cz" means a carbon-based chain that can have from t to z carbon atoms, for example C1-C3 means a carbon-based chain that can have from 1 to 3 carbon atoms.

The term "halogen" means: a fluorine, a chlorine, a bromine or an iodine.

The term "alkyl group" means: a linear or branched, saturated, hydrocarbon-based aliphatic group comprising, unless otherwise mentioned, from 1 to 12 carbon atoms, preferably from 1 to 6 atoms. By way of examples, mention may be made of methyl, ethyl, *n*-propyl, isopropyl, *n*-butyl, isobutyl, *tert*-butyl or *n*-pentyl groups.

The term "alcohol protecting group" means: aryl or arylalkyl, mention may be made of benzyl; Heterocycles, mention may be made of tetrahydrofuranyl, tetrahydropyranyl; C1-C6 alkyl ether mention may be made of *tert*-butyl ethers, C3-C16 silyl ethers, mention may be made of trimethylsilyl, *tert*-butyldimethylsilyl, triisopropylsilyloxymethyl, and triisopropylsilyl ethers or glycosyl.

The term "aryl group" means: a mono or bicyclic aromatic group comprising between 6 and 10 carbon atoms. By way of examples of aryl groups, mention may be made of phenyl or naphthyl groups.

The term "Heteroaryl" means: a 5 to 10 membered aromatic monocyclic or bicyclic group containing from 1 to 4 Heteroatoms selected from N, O, or S. By way of examples, mention may be made of imidazolyl, thiazolyl, oxazolyl, furanyl, thienyl, pyrazolyl, oxadiazolyl, tetrazolyl, pyridinyl, pyrazinyl, pyrimidinyl, pyridazinyl, indolyl, benzofuranyl, benzothienyl, benzoxazolyl, benzimidazolyl, indazolyl, benzothiazolyl, isobenzothiazolyl, benzotriazolyl, quinolinyl and isoquinolinyl groups.

By way of a Heteroaryl comprising 5 to 6 atoms, including 1 to 4 nitrogen atoms, mention may in particular be made of the following representative groups: pyrrolyl, pyrazolyl, 1,2,3-triazolyl, 1,2,4-triazolyl, tetrazolyl and 1,2,3-triazinyl.

Mention may also be made, by way of Heteroaryl, of thienyl, oxazolyl, furazanyl, 1,2,4-thiadiazolyl, naphthyridinyl, quinoxalinyl, phthalazinyl, imidazo[1,2-a]pyridine, imidazo[2,1-b]thiazolyl, cinnolinyl, benzofurazanyl, azaindolyl, benzimidazolyl, benzothienyl, thienopyridyl, thienopyrimidinyl, pyrrolopyridyl, imidazopyridyl, benzoazaindole, 1,2,4 triazinyl, indolizinyl, isoxazolyl, isoquinolinyl, isothiazolyl, purinyl, quinazolinyl, quinolinyl, isoquinolyl, 1,3,4-thiadiazolyl, thiazolyl, isothiazolyl, carbazolyl, and also the corresponding groups resulting from their fusion or from fusion with the phenylgroup.

The term "-C(=O)-R' groups" means an acyl group, wherein R' is chosen from Hydrogen, Alkyl, Halogen, $OR_e$, $NR_fR_g$, Cycloalkyl, and Aryl, wherein $R_e$, $R_f$, $R_g$ identical or different, are independently chosen from H and Alkyl;

The term "cycloalkyl group" means: a mono, bi or tricyclic C3 - C14 carbocyclic ring. By way of examples, mention

may be made of cyclopropyl, cyclobutyl, cyclopentyl, cyclohexyl, etc. groups.

The term "Heterocycle" means: a C3 - C14 membered, saturated or partially unsaturated, monocyclic or bicyclic group comprising from one to three Heteroatoms selected from O, S or N; the Heterocycle group may be attached to the rest of the molecule via a carbon atom or via a Heteroatom; the term bicyclic Heterocycle includes fused bicycles and spiro-type rings.

By way of saturated Heterocycle groups comprising from 3 to 6 atoms, mention may be made of oxiranyl, aziridinyl, oxetanyl, tetrahydrofuranyl, dioxolanyl, pyrrolidinyl, azepinyl, oxazepinyl, pyrazolidinyl, imidazolidinyl, tetrahydrothienyl, dithiolanyl, thiazolidinyl, tetrahydropyranyl, tetrahydropyridinyl, dioxanyl, morpholinyl, piperidinyl, piperazinyl, tetrahydrothiopyranyl, dithianyl, thiomorpholinyl or isoxazolidinyl.

Among the Heterocycle groups, mention may also be made, by way of examples, of bicyclic groups such as (8aR)-hexahydropyrrolo[1,2-a]pyrazin-2(1H)-yl, octahydroindozilinyl, diazepanyl, dihydroimidazopyrazinyl and diazabicycloheptanyl groups, or else diazaspiro rings such as 1,7-diazaspiro[4.4]non-7-yl or 1-ethyl-1,7-diazaspiro[4.4]non-7-yl.

When the Heterocycle groups are substituted, the substitution(s) may be on one (or more) carbon atom(s) and/or on the Heteroatom(s). When Heterocycles comprises several substituents, they may be borne by one and the same atom or different atoms.

The term "alkenyl" refers to a branched or linear monovalent unsaturated aliphatic hydrocarbon group having one or more carbon-carbon double bonds, of 2 to 12 (inclusive) carbon atoms, preferably 2 to 8 (inclusive) carbon atoms, more preferably 2 to 6 (inclusive) carbon atoms. This term is further exemplified by groups as vinyl, propylenyl, butenyl, pentenyl, hexenyl, heptenyl, octenyl, and their linear and branched and isomers.

The term "allenyl" refers to a $-C(R_a)=C=CR_bR_c$ group. The $R_a$, $R_b$ and $R_c$ groups are as defined above.

The term "alkynyl" refers to a branched or linear monovalent unsaturated aliphatic hydrocarbon group having one or more carbon-carbon triple bonds, of 2 to 12 (inclusive) carbon atoms, preferably 2 to 8 (inclusive) carbon atoms, more preferably 2 to 6 (inclusive) carbon atoms. This term is further exemplified by groups as ethynyl, propynyl, butynyl, pentynyl, hexynyl, heptynyl, octynyl, and their linear and branched and isomers.

The term "alkoxy group" means: an -*O*-alkyl radical where the alkyl group is as previously defined. By way of examples, mention may be made of -*O*-(C1-C4)alkyl groups, and in particular -*O*-methyl, -*O*-ethyl, -*O*-propyl, -*O*-isopropyl, -*O*-butyl, -*O*-isobutyl or *O*-*tert*-butyl group.

The abovementioned Alkyl, Alkenyl, Allenyl, Alkynyl, Cycloalkyl, Heterocycle, Aryl, Heteroaryl may be optionally substituted by one or more groups chosen from the group consisting of: halogen, $OR_a$, $COOR_b$, $NR_cR_d$, CN, $NO_2$, $CF_3$, sulfhydryl, arylalkyl, alkylthio, aryloxy or arylalkoxy.

Preferably, the abovementioned Alkyl, Alkenyl, Allenyl, Alkynyl, Cycloalkyl, Heterocycle, Aryl, Heteroaryl may be optionally substituted by one or more groups chosen from the group consisting of: halogen, $OR_a$, $COOR_b$, $NR_cR_d$, CN, $NO_2$, $CF_3$.

When an alkyl radical is substituted with an aryl group, the term "arylalkyl" or "aralkyl" radical is used. The "arylalkyl" or "aralkyl" radicals are aryl-alkyl- radicals, the aryl and alkyl groups being as defined above. Among the arylalkyl radicals, mention may in particular be made of the benzyl or phenethyl radicals.

The term "alkylthio" means: an -S-alkyl group, the alkyl group being as defined above.

The term "aryloxy" means: an -O-aryl group, the aryl group being as defined above.

The term "arylalkoxy" means: an aryl-alkoxy- group with structures of aryl-O-alkyl-, the aryl and alkyl groups being as defined above.

The term "hydroxyl" means: a OH group.

The term "sulfhydryl" means: a SH group

The term "oxo" means: "=O".

According to an embodiment,

- Alkyl refers to a linear or branched C1 - C6 alkyl group;
- Alcohol protecting group refers to Aryl, Heterocycles, C1 - C6 alkyl ether, C3-C16 silyl, or glycosyl;
- Cycloalkyl refers to a mono, bi or tricyclic C3 - C14 carbocyclic ring;
- Heterocycle refers to a mono, bi or tricyclic C3 - C14 membered saturated or unsaturated ring comprising 1 or more Heteroatoms chosen from N, O and S;
- Alkenyl refers to a linear or branched C2 - C6 alkenyl group comprising one or more double bonds;
- Allenyl group refers to a $-C(R_a)=C=CR_bR_c$ group;
- Alkynyl refers to a linear or branched C2 - C6 alkynyl, comprising 1 or more triple bonds;
- Aryl refers to a mono, or bicyclic aromatic C6 - C10 ring;
- Heteroaryl refers to a 5 to 10 membered mono or bicyclic aromatic ring comprising 1 or more Heteroatoms chosen from N, O and S; and/or
- Halogen is chosen from F, Cl, Br and I.

In some embodiments of the invention, the compounds of the invention contain more than one asymmetric center and thus occur as diastereoisomeric mixtures, racemic mixtures, individual diastereoisomeric mixtures or single enantiomers. All such isomeric forms of these compounds are included in the present invention, unless expressly provided otherwise.

For example, in the compounds of the invention, the carbon atom carrying the $R_2$ group is an asymmetric carbon that has either a configuration *R* or a configuration *S*.

For example, in the compounds of the invention, the carbon atom carrying the $R_3$ and $R_4$ group is an asymmetric carbon that has either a configuration *R* or a configuration *S*.

For example, in the compounds of the invention, the carbon atom carrying the $R_5$ and $R_6$ group is an asymmetric carbon that has either a configuration *R* or a configuration *S*.

In some embodiments, the compounds of the invention can take configurations illustrated in formula (II):

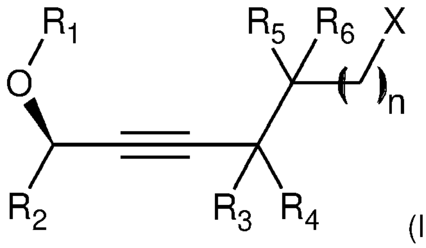

(II)

or a pharmaceutically acceptable salt or ester thereof.

In some embodiments, the compounds of the invention contain one or more double bonds and thus occur as individual or mixtures of *Z* and/or *E* isomers. All such isomeric forms of these compounds are included in the present invention, unless expressly provided otherwise.

In the embodiments where the compounds of the invention contain multiple tautomeric forms, the present invention also includes all tautomeric forms of said compounds unless expressly provided otherwise.

In some embodiments of the invention, the compounds of the invention are in the form of salts. By way of examples, mention may be made of carbonate, phosphate, and sulfate. They can be in non-ionized or in ionized forms.

According to an embodiment of the compounds of the invention, $R_1$ is Hydrogen. This specific family consists of compounds having the following formula (I-1):

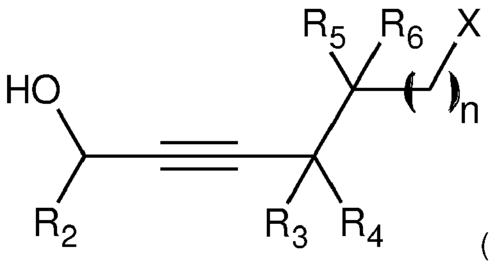

(I-1)

wherein n, $R_2$, $R_3$, $R_4$, $R_5$, and $R_6$, are as defined above in formula (I) or (I').

According to an embodiment, of the compounds of the invention, $R_3$, $R_4$, $R_5$ and $R_6$ identical or different are each independently chosen from the group consisting of Hydrogen and Hydroxyl, wherein at least one of $R_3$, $R_4$, $R_5$ and $R_6$ is Hydroxyl.

According to an embodiment, a specific group of compounds according to the invention may have the following formula (I-2), or (I-3):

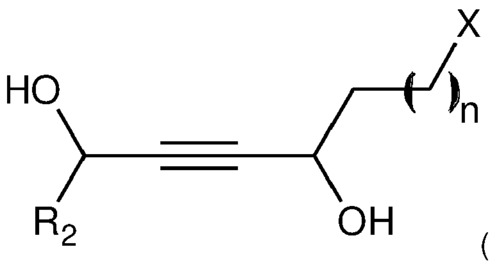

(I-2)

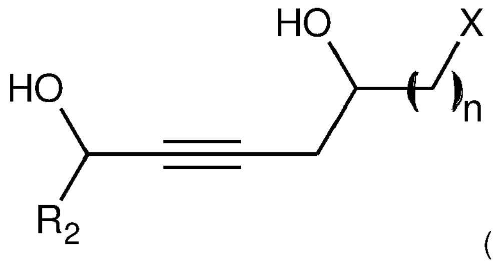

(I-3)

wherein n and $R_2$ are as defined above in formula (I).

According to an embodiment of the compounds of the invention as defined above, $R_2$ is selected from Alkyl, Alkynyl, Cycloalkyl, Aryl and Heteroaryl.

According to an embodiment of the compounds of the invention as defined above, X is selected from $-CH_3$, $-COOH$, $-CH_2OH$, $-NH_2$, Alkynyl, $-CH_2F$, $-CHF_2$, and $-CF_3$.

According to an embodiment of the compounds of the invention, n is comprised between 6 and 12, preferably n is 9 or 10.

The present invention particularly relates to the following compounds of the invention:

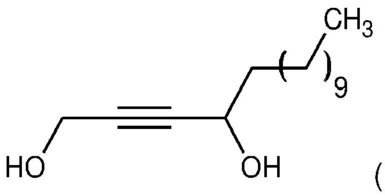

(1)

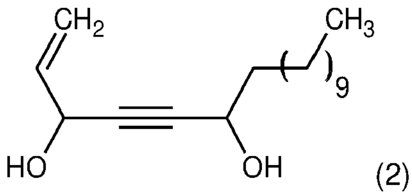

(2)

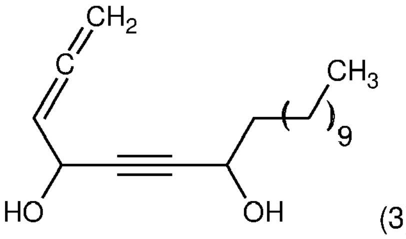

(3)

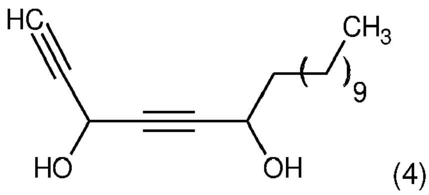

(4)

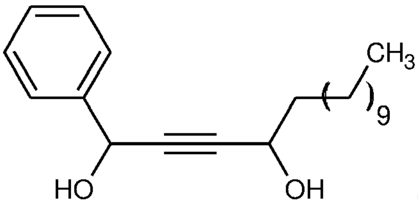

(5)

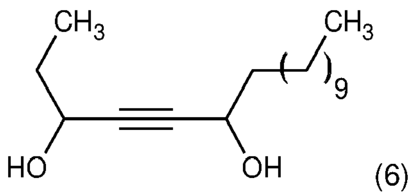

(6)

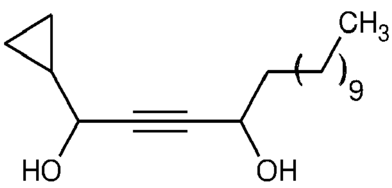

(7)

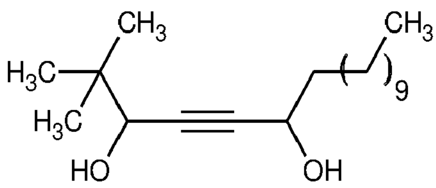

(8)

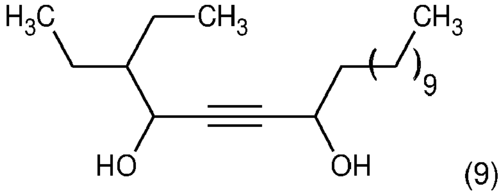

(9)

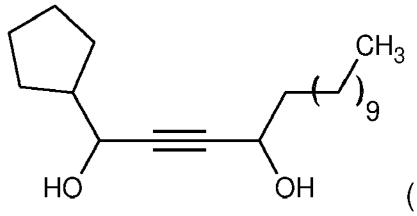

(10)

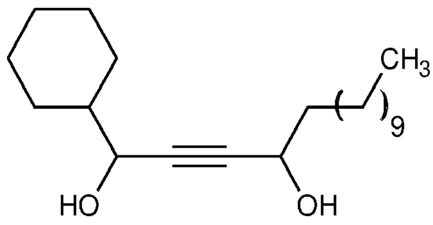
(11)

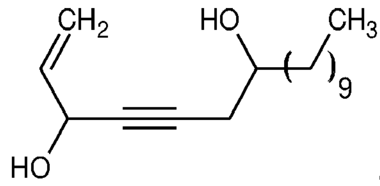
(12)

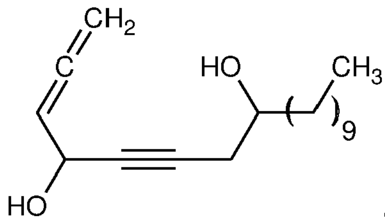
(13)

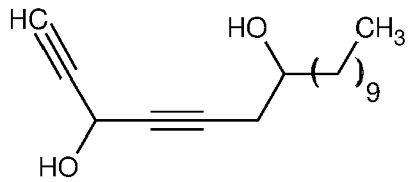
(14)

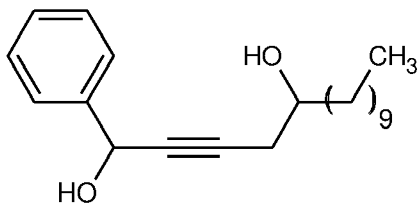
(15)

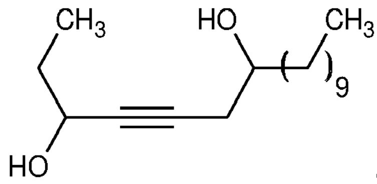
(16)

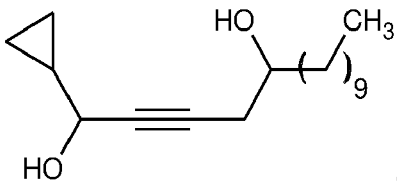
(17)

as well as the stereoisomers thereof,
or a pharmaceutically acceptable salt or ester thereof.

As used herein, a compound of the invention refers to any of formula (I), (I'), (I-1), (I-2), (I-3) and (II), and the pharmaceutically acceptable salts and esters thereof.

The present invention also relates to a process of preparation of a compound of the invention, said process comprising a step of condensation reaction between a metal acetylide (D):

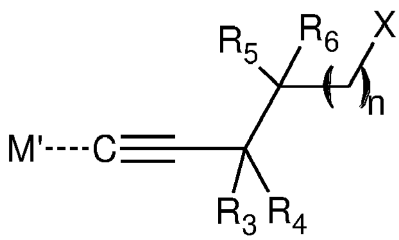
(D)

wherein M' represents an alkali metal based ion, in particular a Lithium ion or a Magnesium bromide ion, and the dashed bond between the M' and the carbon atom represents a covalent bond and/or an ionic bond; and a precursor (C):

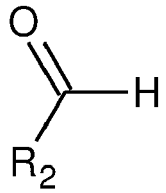

(C)

leading to compound (I-1) of formula:

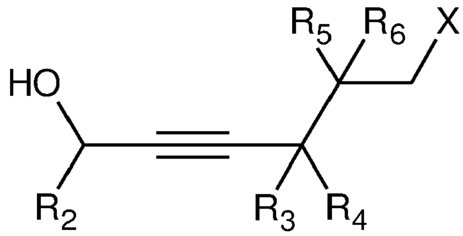

(I-1)

optionally when R1 is different from H by the reaction between the compound (I-1) and a precursor (B):

$R_1$-Z (B)

wherein Z refers to Halogen, or mesylate group ($CH_3$-$SO_2$-O-), or tosylate group (4-$CH_3$-$C_6H_4$-$SO_2$-O-), leading to compound of formula (I):

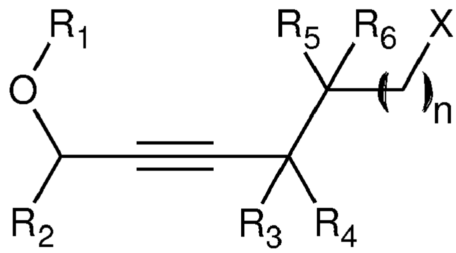

(I)

Wherein $R_1$, $R_2$, $R_3$, $R_4$, $R_5$, $R_6$ and X are defined above.

The process may be carried out by application or adaptation of the illustrative procedures given in the examples 1 to 5 and 14 to 27 stated below.

All reagents (including B and C) may be obtained from commercial suppliers.

A general process of preparation may include: firstly, reagents in an organic solvent solution may be mixed under inert atmosphere such as argon or nitrogen gas at a low temperature between -100 and 0 °C and then may be stirred for around 12 to 20 hours at a temperature ranging from 0 to 40 °C.

In some embodiments, the reactions can be stopped and followed by extraction. Then the product is dried and the volatiles can be removed under reduced pressure (below 1 bar). If necessary, the crude product can be subjected to flash column chromatography.

The present invention also relates to a medicament comprising a compound of formula (I) or formula (I') as defined above.

The present invention also relates to a pharmaceutical composition comprising a compound of formula (I), as defined above, to gather with at least one pharmaceutically acceptable carrier.

While it is possible for the compounds of the invention to be administered alone it is preferred to present them as pharmaceutical compositions. The pharmaceutical compositions, both for veterinary and for human use, useful according to the present invention comprise at least one compound of formula (I), as above defined, together with one or more pharmaceutically acceptable carriers and optionally other therapeutic ingredients.

In certain preferred embodiments, active ingredients necessary in combination therapy may be combined in a single pharmaceutical composition for simultaneous administration.

As used herein, the term "pharmaceutically acceptable" and grammatical variations thereof, as they refer to compositions, salts, esters, carriers, diluents and reagents, are used interchangeably and represent that the materials are capable of administration to or upon a mammal without the production of substantial undesirable physiological effects.

The preparation of a pharmacological composition that contains active ingredients dissolved or dispersed therein is well understood in the art and need not be limited based on formulation. Typically, such compositions are prepared as injectables either as liquid solutions or suspensions; however, solid forms suitable for solution, or suspensions, in liquid prior to use can also be prepared. The preparation can also be emulsified. In particular, the pharmaceutical compositions may be formulated in solid dosage form, for example capsules, tablets, pills, powders, dragees or granules.

The choice of vehicle and the content of active substance in the vehicle are generally determined in accordance with the solubility and chemical properties of the active compound, the particular mode of administration and the provisions to be observed in pharmaceutical practice. For example, excipients such as lactose, sodium citrate, calcium carbonate, dicalcium phosphate and disintegrating agents such as starch, alginic acids and certain complex silicates combined with lubricants such as magnesium stearate, sodium lauryl sulphate and talc may be used for preparing tablets. To prepare a capsule, it is advantageous to use lactose and high molecular weight polyethylene glycols. When aqueous suspensions are used they can contain emulsifying agents or agents which facilitate suspension. Diluents such as sucrose, ethanol, polyethylene glycol, propylene glycol, glycerol and chloroform or mixtures thereof may also be used.

The pharmaceutical compositions can be administered in a suitable formulation to humans and animals by topical or systemic administration, including oral, rectal, nasal, buccal, ocular, sublingual, transdermal, topical, vaginal, parenteral (including subcutaneous, intraarterial, intramuscular, intravenous, intradermal, intrathecal and epidural), intracisternal and intraperitoneal. It will be appreciated that the preferred route may vary with for example the condition of the recipient.

The formulations can be prepared in unit dosage form by any of the methods well known in the art of pharmacy. Such methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then, if necessary, shaping the product.

Total daily dose of the compounds of the invention administered to a subject in single or divided doses may be in amounts, for example, of from about 0.001 to about 100 mg/kg body weight daily and preferably 0.01 to 10 mg/kg/day. Dosage unit compositions may contain such amounts of such submultiples thereof as may be used to make up the daily dose. It will be understood, however, that the specific dose level for any particular patient will depend upon a variety of factors including the body weight, general health, sex, diet, time and route of administration, rates of absorption and excretion, combination with other drugs and the severity of the particular disease being treated.

The present invention also relates to a compound of the invention as defined above and/or the pharmaceutically acceptable salt thereof for use as a medicament.

According to a further object, the present invention relates to use of the compounds of the invention as antibiotic agents.

The present invention also relates to a compound of formula (I") for use as an antibiotic agent:

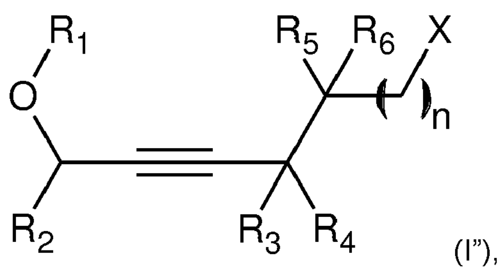

(I"),

wherein:

- n is an integer comprised between 4 and 15;
- $R_1$ is Hydrogen, or a -C(=O)-R' or an alcohol protecting group;
- $R_2$ is chosen from the group consisting of: Hydrogen, Alkyl, Cycloalkyl, Heterocycle, Alkenyl, Allenyl, Alkynyl, Aryl, and Heteroaryl;
- $R_3$, $R_4$, $R_5$ and $R_6$ identical or different are independently chosen from the group consisting of Hydrogen, Halogen, Alkyl, Alkenyl, Alkynyl, AlkylAryl, $OR_a$, $COOR_b$, $NR_cR_d$;
- Alternatively, groups $R_3$ and $R_4$ or groups $R_5$ and $R_6$ can form double carbon-oxygen bond (C=O);
- X is selected from the group consisting of $CH_3$, $CH_2F$, $CHF_2$, $CHFCH_2F$, $CF_3$, COOH, $CH_2OH$, alkoxy, $CCSi(i\text{-}Pr)_3$, alkynyl, $NH_2$, -O-P(=O)($O_2$)M and -O-S(=O)$_2$-OM;

wherein M, $R_a$, $R_b$, $R_c$, $R_d$ and R' are defined as in formula (I);

as well as the stereoisomers thereof,
or a pharmaceutically acceptable salt or ester thereof.

According to an embodiment, in formula (I"), n, $R_1$, $R_2$, $R_3$, $R_4$, $R_5$, $R_6$ and X are defined as in formula (I) or (I') above.
According to an embodiment, compounds of formula (I") are of formula (I) or (I'), as defined above.
According to an embodiment, a compound of formula (I"), having following formula, (I"-1) or (I"-2):

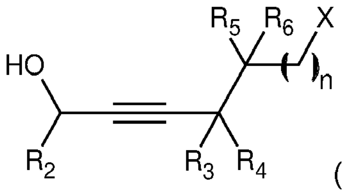
(I"-1),

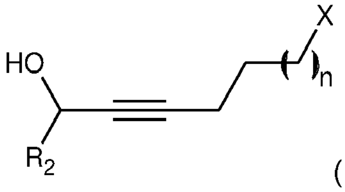
(I"-2),

wherein n, $R_2$, $R_3$, $R_4$, $R_5$, $R_6$ and X are defined as in formula (I) or (I') above.
According to an embodiment, the compound of formula (I") may be chosen from the following compounds:

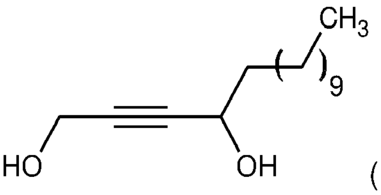
(1)

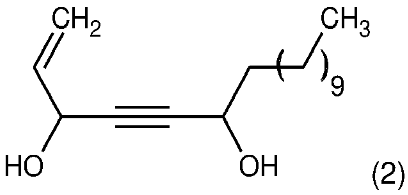
(2)

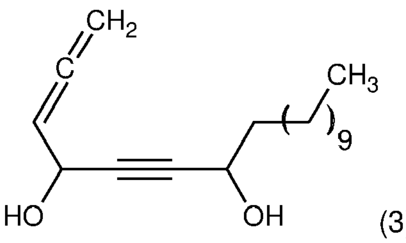
(3)

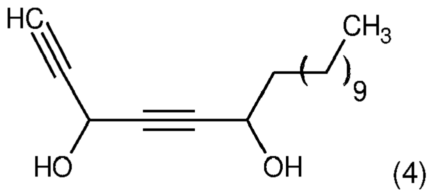
(4)

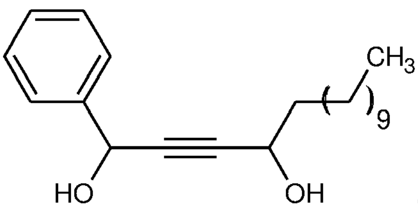
(5)

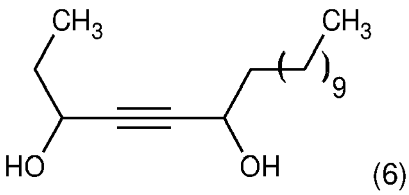
(6)

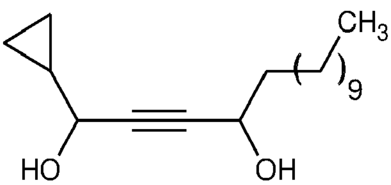
(7)

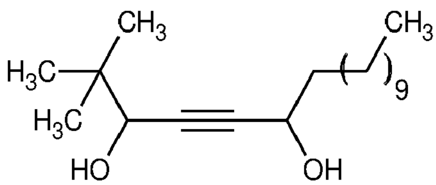
(8)

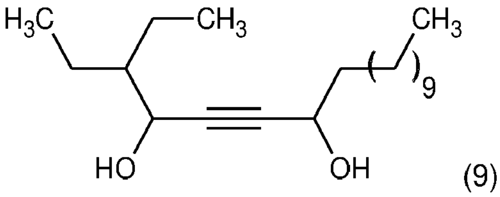
(9)

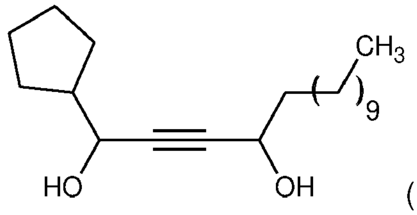
(10)

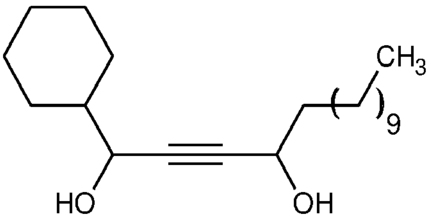
(11)

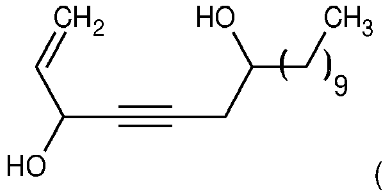
(12)

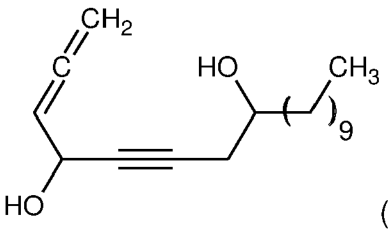
(13)

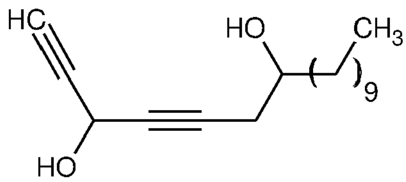
(14)

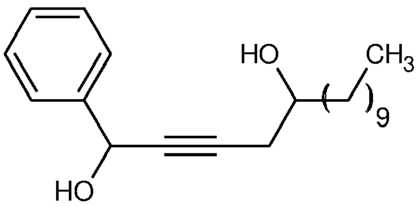
(15)

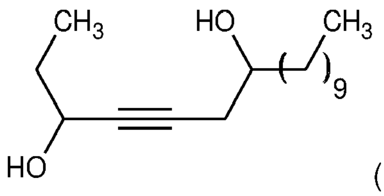
(16)

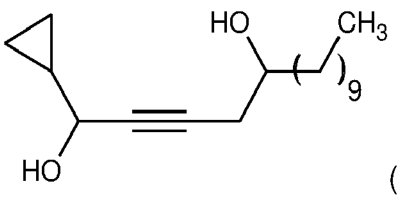
(17)

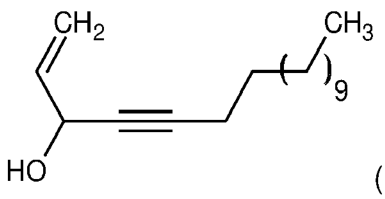
(18)

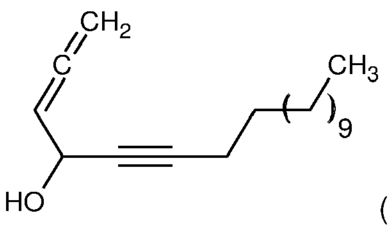
(19)

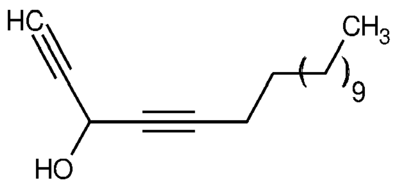
(20)

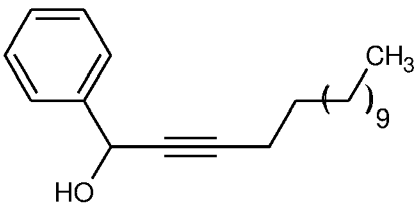
(21)

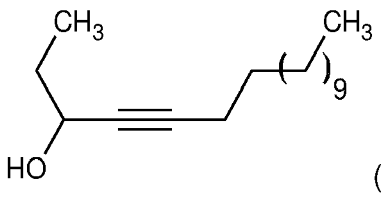
(22)

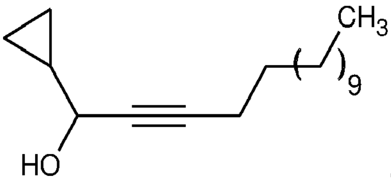
(23)

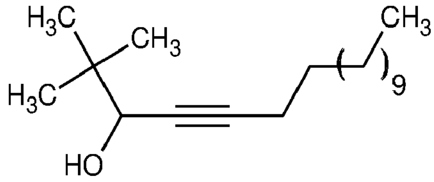
(24)

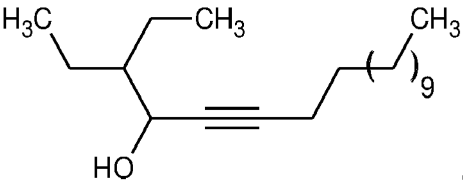
(25)

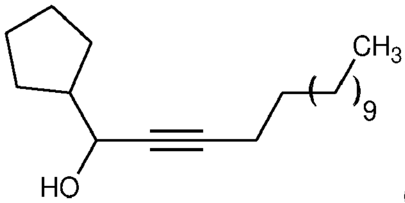
(26)

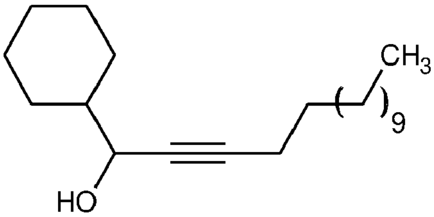
(27)

as well as the stereoisomers thereof,
or a pharmaceutically acceptable salt or ester thereof.

As used herein, a compound of formula (I") refers in particular to any of formula (I), (I'), (II), (I-1), (I-2), (I-3), (I"-1) and (I"-2), and the pharmaceutically acceptable salts and esters thereof.

The present invention also relates to a compound of formula (I") as defined above for use in the treatment of infections caused by bacteria, parasites or fungus.

The present invention also relates to a compound of formula (I") as defined above for use in the treatment of infections caused by bacteria.

According to an embodiment, the said bacterial infections according to the invention are chosen from infections caused by multi-drug resistant (MDR) bacteria, extensively drug-resistant (XDR) bacteria or pandrug-resistant (PDR) bacteria, derived from the above bacteria.

According to an embodiment, the bacterial infections are selected from the group consisting of the following infections: bone infections, lymph nodes infections, respiratory infections, stomach infections, gastrointestinal infections, blood infections, skin infections, bladder infections, kidney infections, urinary tract infections, ear infections, eye infections, and meningeal infections. A skin infection may include an infection of a mucosal membrane, such as the oral cavity, esophagus or eye, e.g. cornea.

According to an embodiment, said bacterial infections are caused by *Mycobacterium tuberculosis, Mycobacterium smegmatis, Mycobacterium bovis, Mycobacterium abscessus, Mycobacterium kansasii, Mycobacterium avium, Escherichia coli, Staphylococcus aureus, Staphylococcus saprophyticus, Klebsiella pneumoniae* and *Corynebacterium glutamicum.*

According to an embodiment, said parasitic infections are caused by parasites selected from the group consisting of: malaria, leishmaniasis, trypanosomiasis, chagas disease, cryptosporidiosis, fascioliasis, filariasis, amebic infections, giardiasis, pinworm infection, schistosomiasis, taeniasis, toxoplasmosis and trichinellosis.

According to an embodiment, said fungal infections are caused by fungus selected from the group consisting of: candidiasis, aspergillosis, coccidioidomycosis, cryptococcosis, histoplasmosis and tinea pedis.

The present invention also relates to a compound of formula (I") as defined above for use as an antibacterial agent.

The present invention also relates to a compound of formula (I") as defined above for use for the treatment of bacterial infections.

According to an embodiment, the bacterial infections are caused by mycobacterial pathogens.

According to an embodiment, said bacterial infections are caused by the *Mycobacterium tuberculosis* complex, in particular, *Mycobacterium tuberculosis* and/or *Mycobacterium bovis.*

According to an embodiment, said bacterial infections are caused by Non-Tuberculous Mycobacteria, in particular, *Mycobacterium abscessus, Mycobacterium smegmatis, Mycobacterium kansasii and/or Mycobacterium avium.*

In the context of the invention, the term "treating" or "treatment", as used herein, means reversing, alleviating, inhibiting the progress of, or preventing the disorder or condition to which such term applies, or one or more symptoms of such disorder or condition.

The present invention also relates to a compound of formula (I") as defined above, for use for further functionalization. Functional groups refer to, but are not limited to, a fluorophore, a biotin, a peptide, a drug or a "clickable" group, such as a C2-alkynyl group or an azido group. The said functional group can be added anywhere on the compound structure of the present invention, providing that it does not interfere with its bioactivity.

The present invention also relates to a compound of formula (I") as defined above and/or for use as a prodrug.

In the case of compounds functionalized with clickable function, the use of "click-chemistry" reactions, such as the copper-catalysed azide-alkyne cycloaddition (CuAAC), allows to subsequently modify the protein with another functional group, including large functional groups, providing that it carries a function amenable to the selected "click chemistry" reaction. The protein of interest can be any protein with lysine and/or cysteine residues, but is preferably an antibody, and the functional group is preferentially a drug (i.e. an antibody-drug conjugates).

Compared to alkynylcarbinols, the oxidized alkynylcarbinones are much less stable in biological environment and can lose rapidly their bioactivities, which explains their low cytotoxicity when exposed directly in media of cells or bacteria. The alkynylcarbinols are thus designed as prodrugs that can be used as antibiotics in medical treatment.

**Preparation of compounds of the present invention:**

**General methods:**

All reagents were obtained from commercial suppliers and used without any further purification. If not specified, reactions were run under nitrogen or argon atmosphere in oven-dried glassware. Standard inert atmosphere techniques were used in handling all air and moisture sensitive reagents. Toluene, dichloromethane (DCM), tetrahydrofuran (THF), dimethylformamide (DMF) and diethyl ether ($Et_2O$) were obtained by filtration through a drying column on a filtration system. Thin-layer chromatography (TLC) analyses were performed on precoated, aluminum-backed silica gel (Merck 60 F254). Visualization of the developed chromatogram was performed by UV light (254 nm) and using 10% phosphomolybdic acid in EtOH, or aqueous potassium permanganate ($KMnO_4$) staining. Flash chromatography columns were performed using flash silica gel (SDS 35-70 $\mu$m). Nuclear magnetic resonance spectra were recorded on a Bruker Advance 300, 400, or 500 MHz spectrometer. Chemical shifts of $^1$H NMR spectra are given in parts per million (ppm) with respect to the residual solvent resonance as the reference $CHCl_3$ ($\delta$ = 7.26 ppm). Data are reported as follows: chemical shift, multiplicity (s = singlet, d = doublet, t = triplet, q = quartet, quint = quintet, m = multiplet and br = broad), coupling constant in Hz and integration. Chemical shifts for $^{13}$C NMR spectra are given in ppm using the central peak of $CDCl_3$ ($\delta$ = 77.16 ppm) as the reference. All $^{13}$C NMR spectra were recorded under complete proton decoupling. High-resolution mass spectrometry (HRMS) was performed on a Thermo-Finnigan MAT 95 XL instrument. Mass spectrometry *m/z*values are given in Dalton units. Optical rotations were measured on a Jasco P-2000 polarimeter. $[\alpha]_D$ Values are given in deg.dm$^{-1}$.cm$^3$.g$^{-1}$.

The compounds of formula (I), (I') or (I") of the present invention were prepared according to one of the following methods:

Compounds of formula (I") were prepared by the general procedure (A):

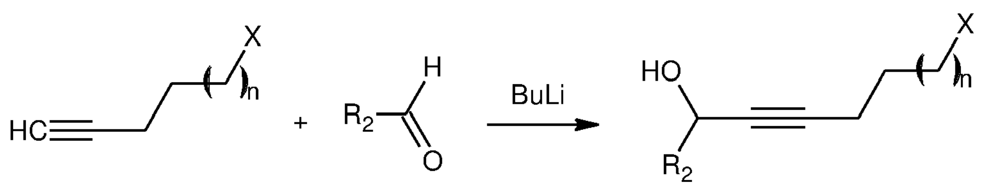


wherein n, $R_2$ and X are defined above, in particular, n = 10, X = H, n-BuLi refers to *n*-Butyllithium.

To a solution of tetradecyne (n = 10, X = H, 0.1 mol/L) in anhydrous THF at -78°C under nitrogen atmosphere, *n*-butyllithium solution (2.5 mol/L in hexane, 1.1 eq.) was added dropwise. After 30 min, a solution of the aldehyde (1 eq., 2.5 mol/L) in anhydrous THF was added dropwise. The reaction was warmed up to room temperature and maintained under stirring overnight. The reaction mixture was quenched by the addition of saturated $NH_4Cl$ aqueous solution and extracted with EtOAc (3 times). The combined extract was dried over $MgSO_4$ and the volatiles were removed under reduced pressure. The crude product was subjected to flash column chromatography (silica gel using a gradient of EtOAc in

pentane) to afford the compounds of formula (I").

Compounds of formula (I), (I-1) or (I-2) were prepared by the general procedure (B):

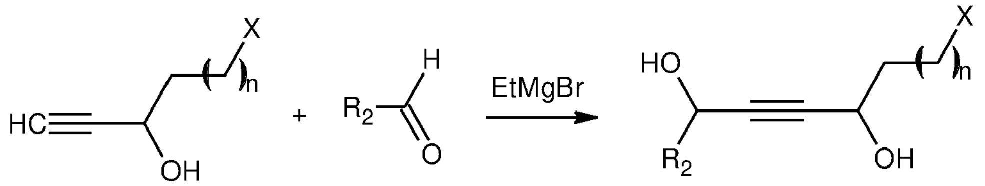


wherein n, $R_2$ and X are defined above, in particular, n = 10, X = H, EtMgBr refers to ethylmagnesium bromide.

To a solution of tetradec-1-yn-3-ol (n = 10, X = H, 0.1 mol/L) in anhydrous THF at 0°C under nitrogen atmosphere, ethyl magnesium bromide solution (3 mol/L in hexane, 2.1 eq.) was added dropwise. After overnight stirring at room temperature, the aldehyde (1.1 eq.) was added dropwise. The mixture was stirred still reaction completion (followed by TLC). The reaction mixture was quenched by the addition of saturated $NH_4Cl$ aqueous solution and extracted with $CH_2Cl_2$ (3 times). The combined extract was washed with saturated $NH_4Cl$ aqueous solution, dried over $MgSO_4$, filtered and the volatiles were removed under reduced pressure. The crude product was subjected to flash column chromatography (silica gel using a gradient of EtOAc in pentane) to afford the compounds of formula (I), (I-1) or (I-2).

Optionally, compounds of formula (I), (I-1) or (I-2) were prepared by the general procedure (C):

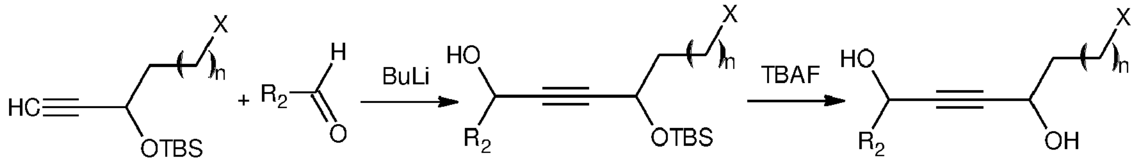


wherein n, $R_2$, X, and *n*-BuLi are defined above, in particular, n = 10, X = H, TBS refers to tert-butyldimethylsilyl, TBAF refers to tetra-*n*-butylammonium fluoride.

To a solution of *tert*-butyldimethyl(tetradec-1-yn-3-yloxy)silane (n = 10, X = H, 0.1 mol/L) in anhydrous THF at -78°C under nitrogen atmosphere, *n*-butyllithium solution (2.5 mol/L in hexane, 1.1 eq.) was added dropwise. After 30 min, a solution of the aldehyde (1 eq., 2.5 mol/L) in anhydrous THF was added dropwise. The reaction was warmed up to room temperature and maintained under stirring overnight. The reaction mixture was quenched by addition of saturated $NH_4Cl$ aqueous solution and extracted with EtOAc (3 times). The combined extract was dried over $MgSO_4$, filtered and the volatiles were removed under reduced pressure. The crude product was subjected to flash column chromatography (silica gel using a gradient of EtOAc in pentane) to afford the 4-silylated-2-alkyn-1,4-diol. This monoprotected diol was the subjected to a desilylation step.

To a stirring solution of 4-silylated-2-alkyn-1,4-diol (0.03 mol/L) in anhydrous THF at 0 °C and under nitrogen atmosphere, was added a solution of tetra-*n*-butylammonium fluoride (1 mol/L in THF, 2.0 eq.). The mixture was stirred until reaction completion (followed by TLC). The reaction was quenched by addition of saturated aqueous solution of $NH_4Cl$, followed by extraction with EtOAc (3 times). The combined organic fractions were dried over anhydrous $MgSO_4$, filtered and the volatiles were removed under reduced pressure. The crude product was subjected to flash column chromatography (silica gel using a gradient of EtOAc in pentane) to afford the compounds of formula (I), (I-1) or (I-2).

Compounds of formula (I), (I-1) or (I-3) were prepared by the general procedure (D):

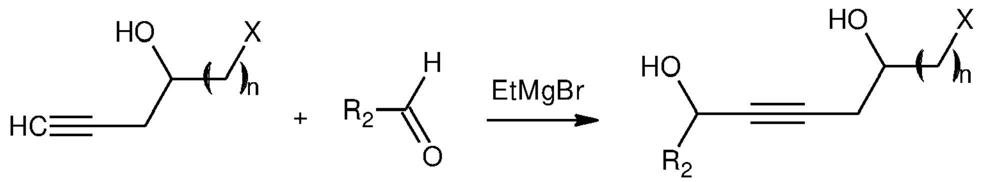


wherein n, $R_2$, X, and EtMgBr are defined above, in particular, n = 10, X = H.

To a solution of tetradec-1-yn-4-ol (n = 10, X = H, 0.1 mol/L) in anhydrous THF at 0°C under nitrogen atmosphere, ethyl magnesium bromide solution (3 mol/L in hexane, 2.1 eq.) was added dropwise. After overnight stirring at room temperature, the aldehyde (1.1 eq.) was added dropwise. The mixture was stirred still reaction completion (followed by TLC). The reaction mixture was quenched by the addition of saturated $NH_4Cl$ aqueous solution and extracted with $CH_2Cl_2$ (3 times). The combined extract was washed with saturated $NH_4Cl$ aqueous solution, dried over $MgSO_4$, filtered and the volatiles were removed under reduced pressure. The crude product was subjected to flash column chromatography (silica

gel using a gradient of EtOAc in pentane) to afford the compounds of formula (I), (I-1) or (I-3).

Optionally, compounds of formula (I), (I-1) or (I-3) were prepared by the general procedure (E):

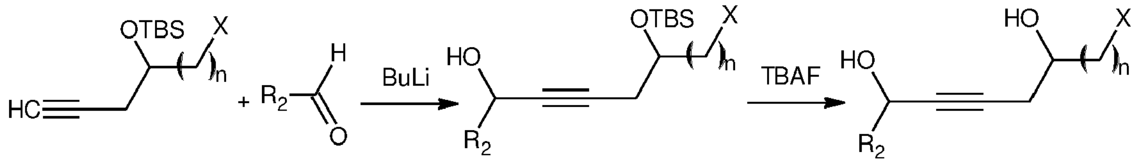


wherein n, $R_2$, X, and *n*-BuLi are defined above, in particular, n = 10, X = H, TBS refers to tert-butyldimethylsilyl, TBAF refers to tetra-*n*-butylammonium fluoride.

To a solution of *tert*-butyldimethyl(tetradec-1-yn-4-yloxy)silane (n = 10, X = H, 0.1 mol/L) in anhydrous THF at -78°C under nitrogen atmosphere, *n*-butyllithium solution (1.6 mol/L in hexane, 1.5 eq.) was added dropwise. After 30 min, the aldehyde (1.5 eq.) was added dropwise. The reaction was warmed up to room temperature and maintained under stirring overnight. The reaction mixture was quenched by the addition of saturated $NH_4Cl$ aqueous solution and extracted with EtOAc (3 times). The combined extract was dried over $MgSO_4$, filtered and the volatiles were removed under reduced pressure. The crude product was subjected to flash column chromatography (silica gel using a gradient of EtOAc in pentane) to afford the 5-silylated-2-alkyn-1,5-diol. This monoprotected diol was the subjected to a desilylation step.

To a stirring solution of 4-silylated-2-alkyn-1,5-diol (0.03 mol/L) in anhydrous THF at 0 °C and under nitrogen atmosphere, was added a solution of tetra-*n*-butylammonium fluoride (1 mol/L in THF, 2.0 eq.). The mixture was stirred until reaction completion (followed by TLC). The reaction was quenched by addition of saturated aqueous solution of $NH_4Cl$, followed by extraction with EtOAc (3 times). The combined organic fractions were dried over anhydrous $MgSO_4$, filtered and the volatiles were removed under reduced pressure. The crude product was subjected to flash column chromatography (silica gel using a gradient of EtOAc in pentane) to afford the compounds of formula (I), (I-1) or (I-3).

**Preparation of enantiomerically enriched compounds of the present invention:**

The crude racemic product synthesized by any one of procedures (A) to (E) was optionally purified by one of the following methods (F) or (G), to obtain enantiomerically enriched compounds:

Method (F): resolution with preparative supercritical fluid chromatography (SFC). Both enantiomers of compounds of formula (I) were obtained via resolution of a racemic mixture by preparative SFC on a Chiralpak IG column.

Method (G): chemoenzymatic resolution:

To a solution of racemic compounds of formula (I) in hexane, vinyl acetate and lipase CALB were added. After reaction, lipase was recycled by filtration and hexane was evaporated under reduced pressure. The mixture was purified by flash chromatography on silica gel to give the (*R*) or (*S*) enantiomer as a white powder and acetylated enriched (*S*) or (*R*) enantiomer as a colorless oil.

**Examples**

Example1: **1-Phenylpentadec-2-yn-1-ol (21):**

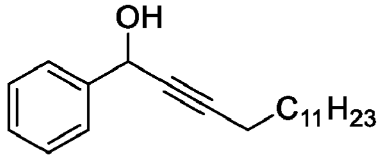

(21)

Following the general procedure A, 1-phenylpentadec-2-yn-1-ol (**21**) (148 mg, 49% yield) was obtained with benzaldehyde.

**Characterization data:** $^{1}$**H NMR** (300 MHz, $CDCl_3$) $\delta$ (ppm) 7.59 - 7.52 (m, 2H), 7.43 - 7.27 (m, 3H), 5.45 (s, 1H), 2.27 (td, $J$ = 7.1, 2.1, 2H), 1.60 - 1.49 (m, 2H), 1.46 - 1.34 (m, 2H), 1.27 (s, 16H), 0.91 - 0.84 (m, 3H). $^{13}$**C NMR** (75 MHz, $CDCl_3$) $\delta$ (ppm) 141.4, 128.7 (2C), 128.3, 126.8 (2C), 87.9, 80.0, 65.0, 32.0, 29.8, 29.8, 29.8, 29.7, 29.5, 29.3, 29.0, 28.7, 22.8, 19.0, 14.2. **HRMS** (DCI-$CH_4$): $m/z$ $[M+H]^+$ calcd for $C_{21}H_{33}O$: 301.2531, found: 301.2524.

Example 2: **2,2-Dimethylheptadec-4-yn-3-ol (24):**

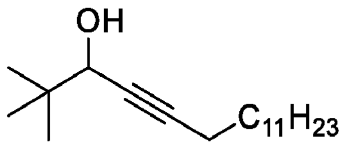

(24)

Following the general procedure A, 2,2-dimethylheptadec-4-yn-3-ol (**24**) (158 mg, 66% yield) was obtained with pivalaldehyde.

**Characterization data: $^1$H NMR** (300 MHz, $CDCl_3$) $\delta$ (ppm) 3.98 (t, $J$= 2.0, 1H), 2.20 (td, $J$ = 7.0, 2.0, 2H), 1.78 (br s, 1H), 1.56 - 1.43 (m, 2H), 1.43 - 1.20 (m, 18H), 0.97 (s, 9H), 0.91 - 0.84 (m, 3H). **$^{13}$C NMR** (75 MHz, $CDCl_3$) $\delta$ (ppm) 86.4, 79.9, 71.7, 35.9, 32.0, 29.8, 29.8, 29.8, 29.7, 29.5, 29.2, 29.0, 28.9, 25.4, 22.8, 18.8, 14.2. **HRMS** (DCI-$CH_4$): *m/z* $[M-H]^+$ calcd for $C_{19}H_{35}O$: 279.2688, found: 279.2693.

Example 3: **3-Ethyloctadec-5-yn-4-ol (25):**

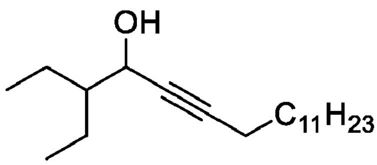

(25)

Following the general procedure A, 3-ethyloctadec-5-yn-4-ol (**25**) (184 mg, 77% yield) was obtained with 2-ethylbutanal.

**Characterization data: $^1$H NMR** (300 MHz, $CDCl_3$) $\delta$ (ppm) 4.39 (br s, 1H), 2.20 (td, $J$ = 7.0, 2.1, 2H), 1.65 - 1.22(m, 26H), 0.97 - 0.84 (m, 10H). **$^{13}$C NMR** (75 MHz, $CDCl_3$) $\delta$ (ppm) 86.4, 80.3, 65.1, 47.8, 32.1, 29.8, 29.8, 29.7, 29.7, 29.5, 29.3, 29.0, 28.9, 22.8, 22.2, 22.0, 18.9, 14.2, 11.8, 11.7. **HRMS** (DCI-$CH_4$): *m/z* $[M-H]^+$ calcd for $C_{20}H_{37}O$: 293.2844, found: 293.2847.

Example 4: **1-Cyclopentylpentadec-2-yn-1-ol (26):**

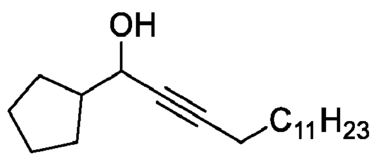

(26)

Following the general procedure A, 1-cyclopentylpentadec-2-yn-1-ol (**26**) (162 mg, 75% yield) was obtained with cyclopentanecarbaldehyde.

**Characterization data: $^1$H NMR** (300 MHz, $CDCl_3$) $\delta$ (ppm) 4.22 (d, $J$ = 6.9, 1H), 2.25 - 2.05 (m, containing at 2.20 (td, $J$= 7.0, 2.0, 2H) and 1H), 1.80 - 1.20 (m, 29H), 0.91 - 0.84 (m, 3H). **$^{13}$C NMR** (75 MHz, $CDCl_3$) $\delta$ (ppm) 85.8, 80.8, 66.8, 46.7, 32.8, 29.8, 29.8, 29.8, 29.7, 29.5, 29.0, 29.0, 28.8, 28.5, 25.9, 22.8, 18.8, 14.2. **HRMS** (DCI-$CH_4$): *m/z*$[M-H]^+$ calcd for $C_{20}H_{35}O$: 291.2688, found: 291.2698.

Example 5: **1-Cyclohexylpentadec-2-yn-1-ol (27):**

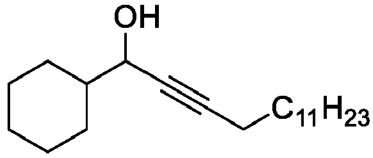 (27)

Following the general procedure A, 1-cyclohexylpentadec-2-yn-1-ol (**27**) (199 mg, 76% yield) was obtained from cyclohexanecarbaldehyde.

**Characterization data:** $^{1}$**H NMR** (300 MHz, $CDCl_3$) $\delta$ (ppm) 4.12 (br s, 1H), 2.19 (td, $J$ = 7.0, 2.3 Hz, 2H), 1.90 - 1.60 (m, 5H), 1.60 - 1.40 (m, 3H), 1.40 - 1.20 (m, 23H), 0.95 - 0.80 (m, 3H). $^{13}$**C NMR** (75 MHz, $CDCl_3$) $\delta$ (ppm) 86.4, 80.8, 67.6, 44.5, 32.0, 29.8, 29.8, 29.7, 29.7, 29.5, 29.2, 29.0, 28.8, 28.7, 28.2, 26.6, 26.1, 26.0, 22.8, 18.8, 14.2. **HRMS** (DCI-$CH_4$): $m/z$ $[M-H]^+$ calcd for $C_{21}H_{37}O$: 305.2844, found: 305.2853.

Example 6: **Heptadec-1-en-4-yn-3-ol (18):**

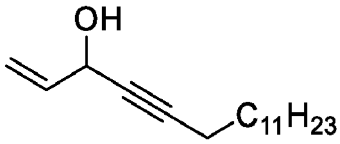 (18)

Following the general procedure A, heptadec-1-en-4-yn-3-ol (**18**) (30 mg, 70% yield) was obtained with freshly distilled acrolein. All analyses were consistent with data reported in the original article (El Arfaoui, D. et al. ChemMedChem 2013, 8, 1779-1786).

Example 7: **Octadeca-1,2-dien-5-yn-4-ol (19):**

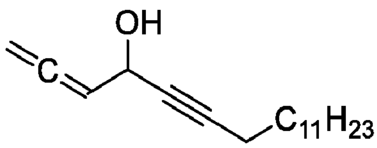 (19)

Octadeca-1,2-dien-5-yn-4-ol (**19**) was prepared according to a previously reported procedure and spectral data matched those reported in the original article (Listunov, D. et al. ChemMedChem 2018, 13, 1711-1722).

Example 8: **Heptadeca-1,4-diyn-3-ol (20):**

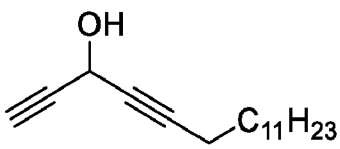 (20)

Heptadeca-1,4-diyn-3-ol (**20**) was prepared according to previously reported procedure and spectral data matched those reported in the original article (Bourkhis, M. et al. ChemMedChem 2018, 13, 1124-1130.).

Example 9: **Heptadec-4-yn-3-ol (22):**

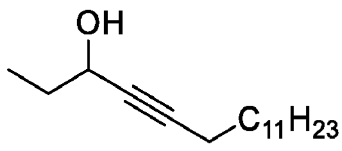
(22)

Heptadec-1-en-4-yn-3-ol (**22**) was prepared according to previously reported procedure and spectral data matched those reported in the original article (Listunov, D. et al. ChemMedChem 2018, 13, 1711-1722.).

Example 10: **1-Cyclopropylpentadec-2-yn-1-ol (23):**

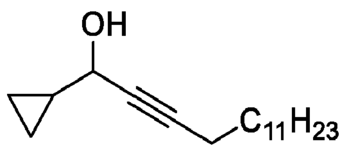
(23)

1-Cyclopropylpentadec-2-yn-1-ol (**23**) was prepared according to previously reported procedure and spectral data matched those reported in the original article (Listunov, D. et al. Tetrahedron 2015, 71, 7920-7930.).

Example 11: **Pentadec-2-yne-1,4-diol (1)** :

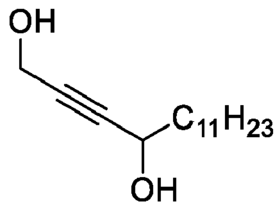
(1)

To a solution of propargylic alcohol (0.25 mL, 4.33 mmol, 1.5 eq.) in anhydrous THF (20 mL) under stirring at - 78 °C was added dropwise under argon *n*-butyllithium (2.5 M in hexane, 3.5 mL, 3 eq.). The mixture is stirred 10 min. at - 78 °C and then 30 min at room temperature. After cooling again at - 78 °C, a solution of dodecanal (0.64 mL, 2.88 mmol, 1 eq.) in anhydrous THF (5 mL) was added dropwise under argon. The reaction mixture was warmed up to room temperature and stirring was maintained overnight before treatment with a saturated aqueous $NH_4Cl$ solution. The aqueous layer was extracted with EtOAc (3 x), and the combined extracts were washed with brine, dried over $MgSO_4$, filtered and concentrated under reduced pressure. The residue was purified by silica gel chromatography ($Et_2O$/pentane 1:1) to afford the pentadec-2-yn-1,4-diol (310 mg, 45 %) as a pale beige solid.

**Characterization data: $^{1}$H NMR** (300 MHz, $CDCl_3$) $\delta$ (ppm) 4.40 (tt, *J* = 6.7, 1.8 Hz, 1H), 4.30 (d, *J*= 1.8 Hz, 2H), 3.59 (br s, 2H), 1.70 (m, 2H), 1.43 (m, 2H), 1.28 (bs, 16H), 0.89 (t, *J*= 6.9 Hz, 3H). **$^{13}$C NMR** (75 MHz, $CDCl_3$) $\delta$ (ppm) 86.8, 83.0; 62.3, 50.7, 37.6, 31.9, 29.7, 29.6 (3s), 29.4, 29.3, 25.2, 22.7, 14.1. **HRMS** (DCI-$CH_4$): *m/z* $[M+H]^+$ calcd for $C_{15}H_{29}O_2$: 241.2168, found: 241.2169.

Separation of enantiomers

Both enantiomers of pentadec-2-yne-1 ,4-diol (**1**) were obtained via resolution of a racemic mixture by preparative SFC on a Chiralpak IG column (5 μm (20 x 250 mm) column, 80:20 $ScCO_2/CH_3CN$, 40 mL/min, 150 bar, 25 °C). Characterization data were identical to those of a racemic sample except for optical rotation.

**(*S*)-Pentadec-2-yne-1,4-diol ((*S*)-1):** 51 mg $[\alpha]_D^{20}$ = -2.5 (c 1.0, $CHCl_3$).

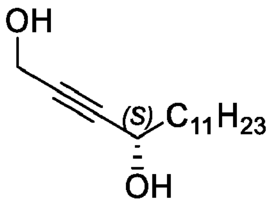

**(*R*)-Pentadec-2-yne-1,4-diol ((*R*)-1):** 53 mg $[\alpha]_D^{20} = +2.7$ (c 1.0, $CHCl_3$).

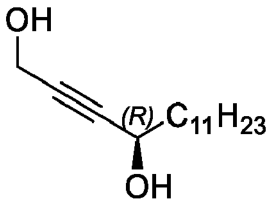

This separation could be run on more than 100 mg and both enantiomers were obtained with high efficiency and purity (> 99% ee, 86% global yield).

Example 12: **Heptadec-1-en-4-yne-3,6-diol (2):**

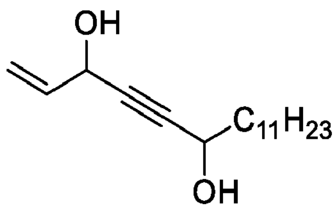
(2)

Following the general procedure B, heptadec-1-en-4-yne-3,6-diol (**2**) (258 mg, 73% yield) was obtained with freshly distilled acrolein.

**Characterization data: $^1$H NMR** (400 MHz, $CDCl_3$) $\delta$ (ppm) 5.97 (ddd, *J*= 17.0, 10.1, 5.3 Hz, 1H), 5.45 (dt, *J*= 17.0, 1.5 Hz, 1H), 5.23 (dt, *J*= 10.2, 1.3 Hz, 1H), 4.95 - 4.93 (m, 1H), 4.42 (t, *J*= 8Hz, 1H), 1.75 - 1.66 (m, 2H), 1.49 - 1.39 (m, 2H), 1.39 - 1.16 (m, 16H), 0.95 - 0.80 (m, 3H). **$^{13}$C NMR** (100 MHz, $CDCl_3$) $\delta$ (ppm) 136.8, 116.6, 87.5, 83.5, 63.1, 62.5, 37.7, 31.9, 29.6, 29.6, 29.6, 29.5, 29.3, 29.2, 25.1, 22.7, 14.1. **HRMS** (DCI-$CH_4$): *m/z* $[M+H]^+$ calcd for $C_{17}H_{31}O_2$: 267.2324, found: 267.2330.

Example 13: **Octadeca-1,2-dien-5-yne-4,7-diol (3):**

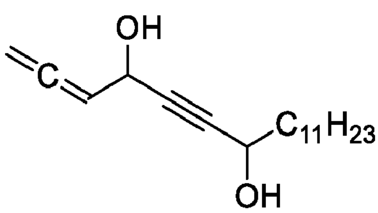
(3)

Paraformaldehyde (8.3 mg, 0.27 mmol, 1.6 eq) and copper (I) iodide (3.3 mg, 0.02 mmol, 0.1 eq) were added in a sealed tube under argon. A solution of diol (46.0 mg, 0.17 mmol) in dry dioxane (1 mL) was added followed by diisopropylamine (35 μL, 0.24 mmol, 1.4 eq). The mixture was stirred at 130 °C overnight. The crude mixture was quenched with a saturated aqueous solution of $NH_4Cl$ (3 mL) and extracted by $Et_2O$ (3 x 5 mL). The organic layer was dried over anhydrous anhydrous $MgSO_4$, filtered and the volatiles were removed under reduced pressure. The crude product

was subjected to flash column chromatography on silica gel (heptane/EtOAc 8 : 2) to give the allene **3** (21 mg, 44% yield).

**Characterization data: $^{1}$H NMR** (300 MHz, $CDCl_3$) $\delta$ (ppm) 5.38 (q, *J* = 7.2, 1H), 5.00 - 4.90 (m, 3H), 4.41 (t, *J* = 6.5, 1H), 2.36 (br s, 1H), 2.11 (br s, 1H), 1.74 - 1.65 (m, 2H), 1.49 - 1.38 (m, 2H), 1.32 - 1.23 (m, 16H), 0.95 - 0.80 (m, 3H). **$^{13}$C NMR** (75 MHz, $CDCl_3$) $\delta$ (ppm) 207.8, 93.1, 87.0, 84.0, 79.0, 62.6, 60.6, 37.8, 32.0, 29.8, 29.8, 29.7, 29.7, 29.5, 29.4, 25.3, 22.8, 14.2. **HRMS** (DCl-$CH_4$): *m/z* [$M^+$] calcd: $C_{18}H_{30}O_2$: 278.2246, found: 278.2260.

Example 14: **Heptadeca-1,4-diyne-3,6-diol (4):**

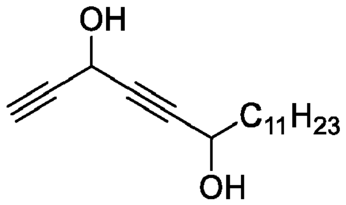

(4)

Following the general procedure C, heptadeca-1,4-diyne-3,6-diol (**4**) (258 mg, 73% yield) was obtained with 3-(trimethylsilyl)-2-propynal.

**Characterization data: $^{1}$H NMR** (300 MHz, $CDCl_3$) $\delta$ (ppm) 5.16 (t, *J*= 1.8, 1H), 4.42 (tt, *J* = 6.6, 1.9, 1H), 3.51 (br s, 1H), 2.87 (br s, 1H), 2.56 (d, *J* = 2.3, 1H), 1.76 - 1.67 (m, 2H), 1.48 - 1.39 (m, 2H), 1.29 - 1.22 (m, 16H), 0.95 - 0.80 (m, 3H). **$^{13}$C NMR** (75 MHz, $CDCl_3$) $\delta$ (ppm) 86.0, 81.7, 80.9, 72.9, 62.5, 51.9, 37.5, 32.0, 29.8, 29.8, 29.7, 29.7, 29.5, 29.4, 25.2, 22.8, 14.2. **HRMS** (DCl-$CH_4$): *m/z* [M-H]$^+$ calcd for $C_{17}H_{29}O_2$: 263.2011, found: 263.2014.

Example 15: **1-Phenylpentadec-2-yne-1,4-diol (5):**

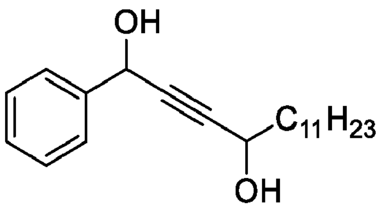

(5)

Following the general procedure C, 1-phenylpentadec-2-yne-1,4-diol (**5**) (87 mg, 44% yield) was obtained with benzaldehyde.

**Characterization data: $^{1}$H NMR** (300 MHz, $CDCl_3$) $\delta$ (ppm) 7.56 - 7.50 (m, 2H), 7.41 - 7.29 (m, 3H), 5.49 (s, 1H), 4.43 (td, *J* = 6.6, 1.7 Hz, 1H), 2.80 (br s, 1H), 2.40 (br s, 1H), 1.78 - 1.64 (m, 2H), 1.50 - 1.36 (m, 2H), 1.36 - 1.17 (m, 16H), 0.95 - 0.80 (m, 3H). **$^{13}$C NMR** (75 MHz, $CDCl_3$) $\delta$ (ppm) 140.6, 128.8 (2C), 128.5, 126.8 (2C), 88.0, 84.7, 64.6, 62.6, 37.8, 32.1, 29.8, 29.8, 29.7, 29.7, 29.5, 29.4, 25.3, 22.8, 14.2. **HRMS** (DCl-$CH_4$): *m/z*[M+H]$^+$ calcd for $C_{21}H_{33}O_2$: 317.2481 found: 317.2480.

Example 16: **Heptadec-4-yne-3,6-diol (6):**

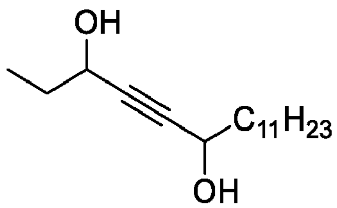

(6)

Following the general procedure C, heptadec-4-yne-3,6-diol (**6**) (31 mg, 38% yield) was obtained with propanal.

**Characterization data: $^1$H NMR** (300 MHz, $CDCl_3$) $\delta$ (ppm) 4.45 - 4.30 (m, 2H), 2.20 (br s, 2H), 1.80 - 1.60 (m, 4H), 1.50 - 1.35 (m, 2H), 1.35 - 1.20 (m, 16H), 1.00 (t, *J* = 7.4, 3H), 0.91 - 0.82 (m, 3H). **$^{13}$C NMR** (75 MHz, $CDCl_3$) $\delta$ (ppm) 86.2, 85.8, 63.8, 62.6, 37.9, 32.1, 31.0, 29.8, 29.8, 29.7, 29.7, 29.5, 29.4, 25.3, 22.8, 14.3, 9.6. **HRMS** (DCI-$CH_4$): *m/z* $[M+H]^+$ calcd for $C_{17}H_{33}O_2$: 269.2481, found: 269.2470.

Example 16-1: separation of enantiomers of **(6*R*)-Heptadec-4-yne-3,6-diol:**

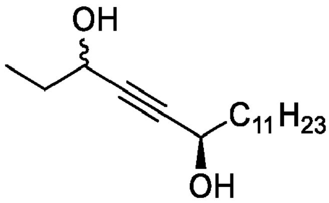

Following the general procedure C, (6*R*)-heptadec-4-yne-3,6-diol (92 mg, 36% yield) was obtained with propanal and (*R*)-*tert*-butyldimethyl(tetradec-1-yn-3-yloxy)silane.

The two diastereoisomers were resolved by a chiral preparative SFC on a Chiralpak IG column (5 μm (20 x 250 mm) column, 70:30 $ScCO_2/CH_3CN$, 50 mL/min, 135 bar, 35 °C). Characterization data were identical to those of a racemic sample except for optical rotation.

**(3*S*,6*R*)-heptadec-4-yne-3,6-diol:** 27 mg $[\alpha]_D^{20}$ = +2.5 (c 1.0, $CHCl_3$).

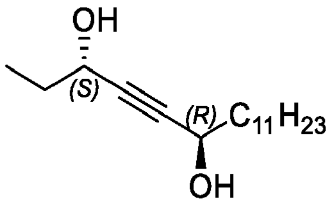

**(3*R*,6*R*)-heptadec-4-yne-3,6-diol:** 25 mg $[\alpha]_D^{20}$ = -5.4 (c 1.0, $CHCl_3$).

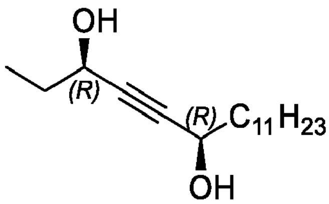

This separation could be run on 92 mg and both enantiomers were obtained with high efficiency and purity (> 99% ee, 58% global yield).

Example 16-2: separation of enantiomers of **(6*S*)-Heptadec-4-yne-3,6-diol:**

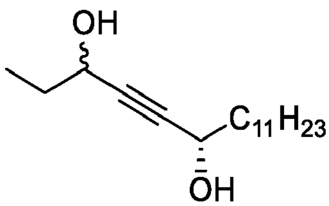

Following the general procedure C, (6*S*)-heptadec-4-yne-3,6-diol (93.8 mg, 37% yield) was obtained with

propanal and (*S*)-*tert*-butyldimethyl(tetradec-1-yn-3-yloxy)silane.

The two diastereoisomers were resolved by a chiral preparative SFC on a Chiralpak IC column (5 μm (20 x 250 mm) column, 80:20 $ScCO_2$/Me, 50 mL/min, 140 bar, 35 °C). Characterization data were identical to those of a racemic sample except for optical rotation.

**(3*S*,6*S*)-heptadec-4-yne-3,6-diol:** 33 mg $[\alpha]_D^{20}$ = +4.7 (c 1.0, $CHCl_3$).

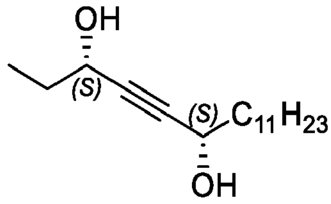

**(3*R*,6*S*)-heptadec-4-yne-3,6-diol:** 28 mg $[\alpha]_D^{20}$ = -5.4 (c 1.0, $CHCl_3$).

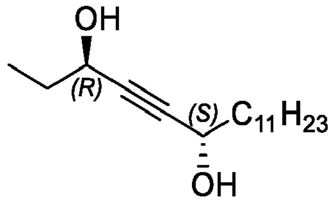

This separation could be run on more 94 mg and both enantiomers were obtained with high efficiency and purity (> 99% ee, 65% global yield).

Optionally, enantiomers of (6*S*)-heptadec-4-yne-3,6-diol were separated by chemoenzymatic resolution:

Preparation of (6*S*)-6-((*tert*-butyldimethylsilyl)oxy)heptadec-4-yn-3-ol:

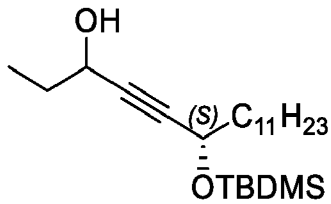

To a solution of (*S*)-*tert*-butyldimethyl(tetradec-1-yn-3-yloxy)silane (311.0 mg, 0.96 mmol) in dry THF (10 mL) under argon and cooled to -78 °C was added *n*-BuLi (1.6 M in hexanes, 0.9 mL, 1.45 eq) dropwise and the mixture was stirred at -78 °C for 1 h. Propanal (100 μL, 1.40 mmol, 1.45 eq, d = 0.810) was added dropwise and the mixture was allowed to warm to room temperature and stirred overnight. The crude mixture was quenched with a saturated solution of $NH_4Cl$ (10 mL) and extracted by EtOAc (3 x 10 mL). The organic layer was dried over anhydrous magnesium sulphate and concentrated under vacuum. The mixture was purified by flash chromatography on silica gel (petroleum ether/EtOAc 95 : 5 and 9 :1) to give the silylated diol (162.0 mg, 44% yield) as a colorless oil (corrected yield 91%).

**Characterization data: $^1$H NMR** (300 MHz, $CDCl_3$): δ = 4.34 (m, 2H), 1.77-1.59 (m, 5H), 1.46-1.35 (m, 2H), 1.32-1.22 (m, 16H,), 1.00 (t, *J* = 7.5, 3H), 0.90 (s, 9H), 0.89-0.85 (m, 3H), 0.12 (s, 3H), 0.10 (s, 3H).

Chemoenzymatic resolution of (6S)-6-((*tert*-butyldimethylsilyl)oxy)heptadec-4-yn-3-ol:

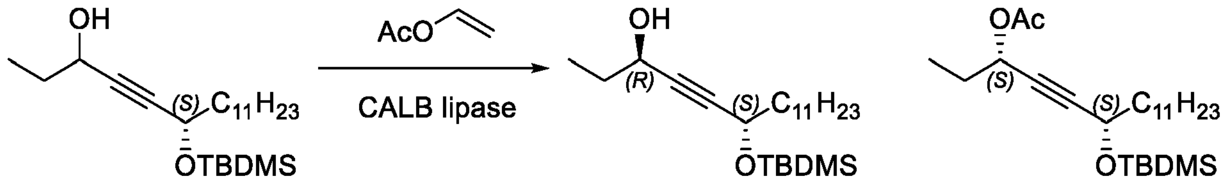


wherein Ac refers to Acetyl, TBDMS refers to *tert*-butyldimethylsilyl.

To a solution of (6*S*)-6-((*tert*-butyldimethylsilyl)oxy)heptadec-4-yn-3-ol (27.0 mg, 0.07 mmol) in hexane (1 mL), vinyl acetate (50 μL, 2.5 eq, d = 0.934) and lipase CALB (5 000 $U.g^{-1}$, 20.0 mg) were added. The reaction mixture was stirred at 40 °C for 16h. Lipase was recycled by filtration and hexane was evaporated under reduced pressure. The mixture was purified by flash chromatography on silica gel (pentane/EtOAc 95 : 5) to give acetylated and silylated (3S,6S)-diol (12.0 mg, 40% yield) and silylated (3R,6S)-diol (11.0 mg, 41%) as colorless oils.

**(3*S*,6*S*)-6-((*tert*-Butyldimethylsilyl)oxy)heptadec-4-yn-3-yl acetate:**

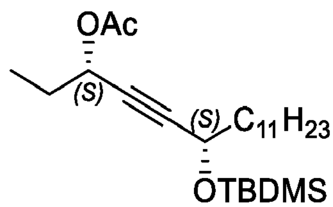

**Characterization data: $^1$H NMR** (300 MHz, $CDCl_3$): δ = 5.33 (td, J = 6.5, 1.6, 1H), 4.35 (td, J = 6.5, 1.5, 1H), 2.06 (s, 3H), 1.80-1.72 (m, 2H), 1.67-1.60 (m, 2H), 1.44-1.36 (m, 2H), 1.29-1.25 (m, 16H), 0.99 (t, J = 7.4 Hz, 3H), 0.89 (s, 9H), 0.89-0.85 (m, 3H), 0.11 (s, 3H), 0.09 (s, 3H). **$^{13}$C NMR** (75 MHz, $CDCl_3$): δ = 170.1, 87.4, 81.2, 65.4, 63.0, 38.6, 32.1, 29.8, 29.8, 29.7, 29.7, 29.5, 29.4, 28.2, 25.4, 22.8, 25.9, 21.1, 18.4, 14.2, 9.5, -4.4, -4.9. **HRMS** (DCI-$CH_4$): *m/z* $[M-H]^+$ calculated : $C_{25}H_{47}O_3Si$ : 423.3294, found : 423.3301.

**Deprotection of silylated (3*R*,6*S*)-heptadec-4-yne-3,6-diol:**

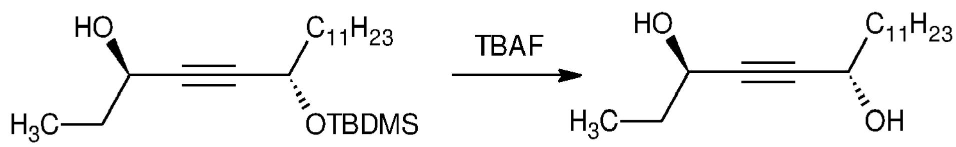


wherein TBAF and TBDMS are defined above.

To a solution of silylated (3*R*,6*S*)-heptadec-4-yne-3,6-diol (11.0 mg, 0.028 mmol) in dry THF (1 mL) under $N_2$ and cooled to 0 °C was added TBAF (1 M in THF, 0.04 mL, 1.4 eq) dropwise and the mixture was stirred at room temperature for 3h. The crude mixture was evaporated under vacuum and purified by flash chromatography on silica gel (pentane/EtOAc 6 : 4) to give (3*R*,6*S*)-diol (7.0 mg, 90% yield, 90% ee) as a white powder.

Example 17: **1-Cyclopropylpentadec-2-yne-1,4-diol (7):**

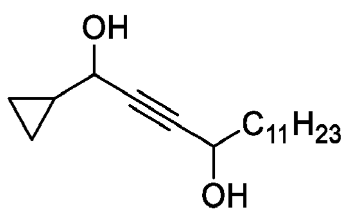

(7)

Following the general procedure B, 1-cyclopropylpentadec-2-yne-1,4-diol (**7**) (111 mg, 87% yield) was obtained with cyclopropanecarbaldehyde.

**Characterization data: $^1$H NMR** (300 MHz, $CDCl_3$) $\delta$ (ppm) 4.38 (tt, *J* = 6.6, 1.7, 1H), 4.24 (dt, *J* = 6.5, 1.6, 1H), 2.33 (br s, 2H), 1.80 - 1.60 (m, 2H), 1.50 - 1.35 (m, 2H), 1.35 - 1.15 (m, 17H), 0.95 - 0.80 (m, 3H), 0.60 - 0.48 (m, 2H), 0.48 - 0.36 (m, 2H). **$^{13}$C NMR** (75 MHz, $CDCl_3$) $\delta$ (ppm) 87.4, 83.7, 65.8, 62.5, 37.8, 32.0, 29.8, 29.8, 29.7, 29.7, 29.5, 29.4, 25.3, 22.8, 17.1, 14.2, 3.4, 1.7. **HRMS** (DCI-$CH_4$): *m/z* $[M+H]^+$ calcd for $C_{18}H_{33}O_2$: 281.2481, found: 281.2484.

Example 18: **2,2-Dimethylheptadec-4-yne-3,6-diol (8):**

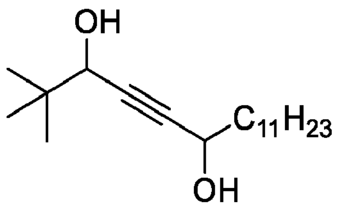

(8)

Following the general procedure B, 2,2-dimethylheptadec-4-yne-3,6-diol (**8**) (115 mg, 31% yield, 64% conversion) was obtained with pivalaldehyde.

**Characterization data: $^1$H NMR** (300 MHz, $CDCl_3$) $\delta$ (ppm) 4.39 (tdd, *J* = 6.6, 2.9, 1.6, 1H), 4.04 (dd, *J* = 3.8, 1.6, 1H), 2.49 (br s, 2H), 1.80 - 1.60 (m, 2H), 1.50 - 1.35 (m, 2H), 1.35 - 1.20 (m, 16H), 0.97 (s, 9H), 0.91 - 0.82 (m, 3H). **$^{13}$C NMR** (75 MHz, $CDCl_3$) $\delta$ (ppm) 87.0, 84.7, 71.4, 62.6, 37.9, 35.9, 32.0, 29.8, 29.8, 29.7, 29.7, 29.5, 29.4, 25.4, 25.3, 22.8, 14.2, 9.6. **HRMS** (DCI-$CH_4$): *m/z* $[M-H]^+$ calcd for $C_{19}H_{35}O_2$: 295.2637, found: 295.2636.

Example 19: **3-Ethyloctadec-5-yne-4,7-diol (9):**

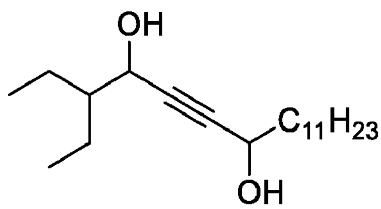

(9)

Following the general procedure C, 3-ethyloctadec-5-yne-4,7-diol (**9**) (36 mg, 37% yield) was obtained with 2-ethylbutanal.

**Characterization data: $^1$H NMR** (400 MHz, $CDCl_3$) $\delta$ (ppm) 4.46 - 4.42 (m, 1H), 4.42 - 4.36 (m, 1H), 1.76 - 1.63 (m, 2H), 1.63 - 1.52 (m, 1H), 1.51 - 1.36 (m, 6H), 1.25 (s, 16H), 0.94 - 0.85 (m, 9H). **$^{13}$C NMR** (100 MHz, $CDCl_3$) $\delta$ (ppm) 86.9/86.9 (diastereoisomers), 85.1/85.1 (diastereoisomers), 64.8, 62.6, 47.5, 37.9/37.9 (diastereoisomers), 32.0, 29.8, 29.8, 29.7, 29.7, 29.5, 29.4, 25.3, 22.8, 22.2/22.1 (diastereoisomers), 21.9, 14.2, 11.7, 11.6. **HRMS** (DCI-$CH_4$): *m/z* $[M+H]^+$ calcd for $C_{20}H_{39}O_2$: 311.2950 found: 311.2958.

Example 20: **1-Cyclopentylpentadec-2-yne-1,4-diol (10):**

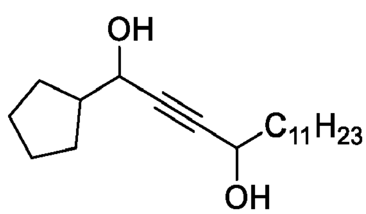

(10)

Following the general procedure C, 1-cyclopentylpentadec-2-yne-1,4-diol (**10**) (11 mg, 10% yield) was obtained with cyclopentanecarbaldehyde.

**Characterization data:** $^{1}$**H NMR** (400 MHz, $CDCl_3$) $\delta$ (ppm) 4.41 (t, J = 8,0 1H), 4.27 (d, J = 7.0, 1H), 2.19 (dt, J = 15.5, 7.9, 1H), 1.82 - 1.72 (m, 2H), 1.70 - 1.55 (m, 6H), 1.49 - 1.39 (m, 4H), 1.26 (s, 16H), 0.91 - 0.82 (m, 3H). $^{13}$**C NMR** (100 MHz, $CDCl_3$) $\delta$ (ppm) 86.2/86.2 (diastereoisomers), 85.5/85.5 (diastereoisomers), 66.5, 62.7, 46.4, 38.0, 32.0, 29.8, 29.8, 29.7, 29.7, 29.5, 29.4, 29.0/29.0 (diastereoisomers), 28.5, 25.9/25.8 (diastereoisomers), 25.3, 22.8, 14.3. **HRMS** (DCI-$CH_4$): *m/z* $[M+H]^+$ calcd for $C_{20}H_{37}O_2$: 309.2794 found: 309.2792.

Example 21: **1-Cyclohexylpentadec-2-yne-1,4-diol (11):**

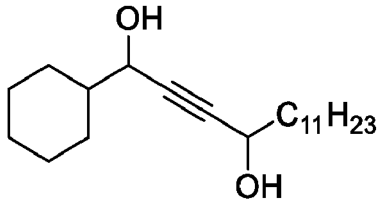

(11)

Following the general procedure B, 1-cyclopropylpentadec-2-yne-1,4-diol (**11**) (139 mg, 43% yield, 52% conversion) was obtained with cyclohexanecarbaldehyde.

**Characterization data:** $^{1}$**H NMR** (300 MHz, $CDCl_3$) $\delta$ (ppm) 4.38 (tt, *J* = 6.6, 2.0, 1H), 4.17 (ddd, *J* = 6.1, 3.3, 1.6, 1H), 2.76 (br s, 2H), 1.90 - 1.60 (m, 5H), 1.60 - 1.35 (m, 3H), 1.35 - 1.20 (m, 23H), 0.95 - 0.80 (m, 3H). $^{13}$**C NMR** (75 MHz, $CDCl_3$) $\delta$ (ppm) 87.0/87.0 (diastereoisomers), 85.0/84.9 (diastereoisomers), 67.2, 62.5, 44.1, 37.9/37.9 (diastereoisomers), 32.0, 29.8, 29.8, 29.7 (2C), 29.5, 29.4, 28.8/28.8 (diastereoisomers), 28.2, 26.5, 26.0, 26.0, 25.4/25.4 (diastereoisomers), 22.8, 14.2. **HRMS** (DCI-$CH_4$): *m/z* $[M-H]^+$ calcd for $C_{21}H_{37}O_2$: 321.2794, found: 321.2794.

Example 22: **Heptadec-1-en-4-yne-3,7-diol (12):**

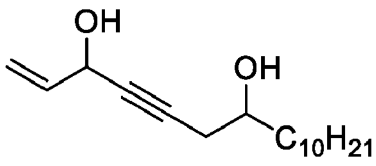

(12)

Following the general procedure D, heptadec-1-en-4-yne-3,7-diol (**12**) (17 mg, 13% yield) was obtained with freshly distilled acrolein.

**Characterization data:** $^{1}$**H NMR** (500 MHz, $CDCl_3$) $\delta$ (ppm) 5.97 (ddd, *J*= 17.0, 10.1, 5.4 Hz, 1H), 5.44 (dt, *J* = 17.0, 1.4 Hz, 1H), 5.21 (dt, *J* = 10.1, 1.3 Hz, 1H), 4.88 (br s, 1H), 3.78 -3.70 (m, 1H), 2.49 (ddd, *J* = 16.7, 4.6, 2.0, 1H), 2.37 (ddd, *J* = 16.7, 6.9, 2.0, 1H), 2.11 (br s, 1H), 1.99 (br s, 1H), 1.56 - 1.46 (m, 2H), 1.46 - 1.36 (m, 2H), 1.36 - 1.13 (m, 14H), 0.91 - 0.84 (m, 3H). $^{13}$**C NMR** (125 MHz, $CDCl_3$) $\delta$ (ppm) 137.3, 116.2, 84.2, 81.5, 70.1, 63.4, 36.4, 31.9, 29.6, 29.6, 29.6, 29.5, 29.3, 27.7, 25.6, 22.7, 14.1. **HRMS** (DCI-$CH_4$): *m/z* $[M-H]^+$ calcd for $C_{17}H_{29}O_2$: 265.2168, found: 265.2154.

Example 23: **Heptadeca-1,4-diyne-3,7-diol (14):**

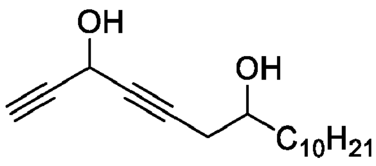

(14)

Following the general procedure D with 3-(trimethylsilyl)-2-propynal, heptadeca-1,4-diyne-3,7-diol (**14**) (87 mg,

35% yield, 57% conversion) was obtained after a $K_2CO_3$ treatment in methanol to remove the trimethylsilyl group.

**Characterization data: $^{1}$H NMR** (300 MHz, $CDCl_3$) $\delta$ (ppm) 5.15 (br s, 1H), 3.81 (p, $J$ = 6.1, 1H), 3.10 - 2.90 (m, 1H), 2.58 (d, $J$ = 2.3, 1H), 2.52 (dddd, $J$ = 16.8, 4.5, 2.1, 1.5, 1H), 2.39 (dddd, $J$ = 16.8, 6.8, 2.1, 1.1, 1H), 2.25 (br s, 1H), 1.65 - 1.50 (m, 2H), 1.50 - 1.20 (m, 16H), 0.95 - 0.85 (m, 3H). **$^{13}$C NMR** (75 MHz, $CDCl_3$) $\delta$ (ppm) 82.4, 81.4, 79.6, 72.5, 70.2/70.1 (diastereoisomers), 52.1, 36.5, 32.0, 29.8 (2C), 29.7, 29.7, 29.5, 27.7, 25.8, 22.8, 14.3. **HRMS** (DCl-$CH_4$): $m/z$ [M+H]$^+$ calcd for $C_{17}H_{29}O_2$: 265.2168, found: 265.2156.

Example 24: **Octadeca-1,2-dien-5-yne-4,8-diol (13):**

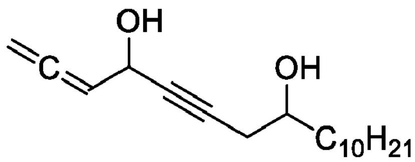
(13)

Paraformaldehyde (5.8 mg, 0.19 mmol, 1.6 eq) and copper (I) iodide (2.3 mg, 1.2 pmol, 0.1 eq) were added in a sealed tube under argon. A solution of the starting diol (32.0 mg, 0.12 mmol) in dry dioxane (0.5 mL) was added followed by diisopropylamine (24 μL, 0.17 mmol, 1.4 eq). The mixture was stirred at 130 °C overnight. The crude mixture was quenched with a saturated aqueous solution of $NH_4Cl$ (3 mL) and extracted by $Et_2O$ (3x5 mL). The organic layer was dried over anhydrous $MgSO_4$, filtered and the volatiles were removed under reduced pressure. The crude product was subjected to flash column chromatography on silica gel (heptane/EtOAc 8 : 2) to give allene **13** (24 mg, 72% yield).

**Characterization data: $^{1}$H NMR** (300 MHz, $CDCl_3$) $\delta$ (ppm) 5.42 (qd, $J$ = 6.3, 0.9, 1H), 5.05 - 4.92 (m, 3H), 3.76 (br s, 1H), 2.50 (ddt, $J$ = 16.7, 4.1, 1.9, 1H), 2.40 - 2.33 (m, containing at 2.37 (ddt, $J$ = 16.7, 7.0, 1.9, 1H) and 1H), 2.16 (br s, 1H), 1.60 - 1.50 (m, 2H), 1.50 - 1.20 (m, 16H), 0.95 - 0.85 (m, 3H). **$^{13}$C NMR** (75 MHz, $CDCl_3$) $\delta$ (ppm) 207.8, 93.6, 83.0, 82.0, 78.8, 70.1/70.1 (diastereoisomers), 60.8/60.8 (diastereoisomers), 36.4, 32.0, 29.8, 29.8, 29.7, 29.7, 29.5, 27.8, 25.8, 22.8, 14.2. **HRMS** (DCl-$CH_4$): $m/z$ [M$^+$] calcd: $C_{18}H_{30}O_2$: 278.2246, found: 278.2254.

Example 25: **1-Phenylpentadec-2-yne-1,5-diol (15):**

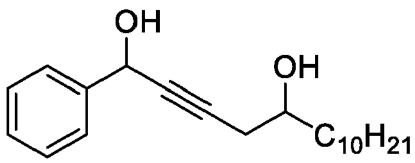
(15)

Following the general procedure E, 1-phenylpentadec-2-yne-1,5-diol (**15**) (32 mg, 22% yield) was obtained from benzaldehyde.

**Characterization data: $^{1}$H NMR** (300 MHz, $CDCl_3$) $\delta$ (ppm) 7.56 - 7.50 (m, 2H), 7.40 - 7.30 (m, 3H), 5.44 (br s, 1H), 3.76 (quint, $J$ = 6.3, 1H), 3.08 (br s, 1H), 2.50 (ddd, $J$ = 16.7, 4.5, 2.1, 1H), 2.37 (ddd, $J$ = 16.7, 6.8, 2.0, 1H), 1.60 - 1.45 (m, 2H), 1.45 - 1.20 (m, 17H), 0.93 - 0.83 (m, 3H). **$^{13}$C NMR** (75 MHz, $CDCl_3$) $\delta$ (ppm) 141.2, 128.7 (2C), 128.4, 126.7 (2C), 84.0, 82.8, 70.2, 64.8, 36.5, 32.0, 29.8 (2C), 29.7, 29.7, 29.5, 27.8, 25.8, 22.8, 14.2. **HRMS** (DCl-$CH_4$): $m/z$[M-H]$^+$ calcd for $C_{21}H_{31}O_2$: 315.2324, found: 315.2338.

Example 26: **Heptadec-4-yne-3,7-diol (16):**

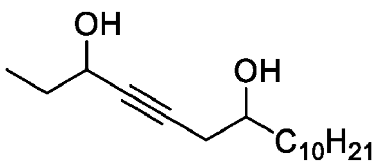
(16)

Following the general procedure E, heptadec-4-yne-3,7-diol (**16**) (43 mg, 34% yield) was obtained from propanal.

**Characterization data: $^{1}$H NMR** (300 MHz, $CDCl_3$) $\delta$ (ppm) 4.55 - 4.25 (m, 1H), 3.80 - 3.65 (m, 1H), 2.73 (br s, 1H), 2.54 (br s, 1H), 2.45 (ddd, *J* = 16.6, 4.5, 2.0, 1H), 2.31 (ddd, *J* = 16.7, 6.8, 2.0, 1H), 1.80 - 1.60 (m, 2H), 1.57 - 1.45 (m, 2H), 1.45 - 1.15 (m, 16H), 0.99 (t, *J*= 7.4, 3H), 0.91 - 0.82 (m, 3H). **$^{13}$C NMR** (75 MHz, $CDCl_3$) $\delta$ (ppm) 83.8, 81.8, 70.2/70.2 (diastereoisomers), 63.9, 36.4/36.4 (diastereoisomers), 32.0, 31.2/31.2 (diastereoisomers), 29.7 (2C), 29.7, 29.7, 29.5, 27.7/27.7 (diastereoisomers), 25.8, 22.8,14.2, 9.6. **HRMS** (DCI-$CH_4$): *m/z* $[M-H]^+$ calcd for $C_{17}H_{31}O_2$: 267.2324, found: 267.2326.

Example 27: **1-Cyclopropylpentadec-2-yne-1,5-diol (17):**

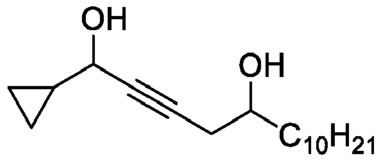

(17)

Following the general procedure D, 1-cyclopropylpentadec-2-yne-1,5-diol (**17**) (70 mg, 52% yield, 80% conversion) was obtained from cyclopropanecarbaldehyde.

**Characterization data: $^{1}$H NMR** (300 MHz, $CDCl_3$) ($\delta$ (ppm) 4.16 (dt, *J* = 6.6, 2.0, 1H), 3.77 -3.65 (m, 1H), 3.06 (br s, 1H), 2.79 (br s, 1H), 2.43 (ddd, *J* = 16.7, 4.4, 2.0, 1H), 2.29 (ddd, *J* = 16.7, 6.8, 2.0, 1H), 1.60 - 1.45 (m, 2H), 1.45 -1.15 (m, 17H), 0.95 - 0.80 (m, 3H), 0.57 - 0.46 (m, 2H), 0.46 - 0.35 (m, 2H). **$^{13}$C NMR** (75 MHz, $CDCl_3$) $\delta$ (ppm) 82.1, 81.6, 70.2/70.2 (diastereoisomers), 65.8, 36.4/36.4 (diastereoisomers), 32.0, 29.7 (2C), 29.7, 29.7, 29.5, 27.7/27.7 (diastereoisomers), 25.8, 22.8, 17.3, 14.2, 3.3, 1.7. **HRMS** (DCI-$CH_4$): *m/z*$[M-H]^+$ calcd for $C_{18}H_{31}O_2$: 263.2375, found: 263.2372.

**Cytotoxicity of compounds of the present invention:**

The biological activity of the compounds of the present invention were evaluated. The activity against mycobacteria (*Mtb*) was assessed by minimum inhibitory concentration values (MIC, μM) determination. The selectivity index (SI) is defined as:

$$SI = \frac{CC_{50}}{MIC}$$

wherein $CC_{50}$ refers to the half maximal cytotoxic concentration of compounds towards particular eukaryotic cells, either for HCT116 cells and/or VERO cells; *MIC* refers to the minimum inhibitory concentration of the compounds towards mycobacteria.

Therefore, a lower anti-mycobacterial (anti-*Mtb*) MIC value indicates higher activity against mycobacteria and a higher SI indicates better selectivity of the compounds.

**Mycobacterial growth inhibition assays**

The sensitivity of *Mycobacterium tuberculosis* strain $H_{37}Rv$ to all synthesized compounds was evaluated using a colorimetric micro assay based on the reduction of MTT (3-(4,5-dimethylthiazol-2-yl)-2,5-diphenyltetrazolium bromide, Sigma) to formazan by metabolically active bacteria. Briefly, serial twofold dilutions of each compound solubilised in DMSO were prepared in 7H9 broth (Difco Middlebrook 7H9 broth, Becton Dickinson and Company, complemented with glycerol (4g/L)) using 96- well microtiter plates, and 100 μL of *M. tuberculosis* H37Rv suspension in 7H9 broth were added to each well (OD600 0.05). After 6 days incubation, MTT was added (50 μL, 1 mg/mL in 7H9 broth). After 24 h incubation, 50 μL of SDS 20% were added to each well. The optical densities were measured at 570 nm with a microplate reader Expert Plus (ASYS HITECH, Austria). The absorbance value for untreated bacilli was set as 100% growth control. The MIC from at least three independent experiments was determined as the lowest concentration of compound that inhibited bacterial growth; dose-response curves based on a non-linear regression were fitted using the GraphPad Prism software.

**Cytotoxicity evaluation on HCT-116 cell line**

Human HCT-116 colon cancer cells were seeded in 96-well plates (2500 cells/well) 24 hours before being treated for 72 hours with the tested compounds. Cells were grown and treated in DMEM 10% FBS. For each compound, 11 concentrations, corresponding to twofold serial dilutions from the maximum concentration, were evaluated, with each concentration tested in duplicate. DMSO was set at 0.5% final concentration in each well, including in the untreated conditions. At the end of the treatment, cells were fixed 1 hour at 4°C by addition of cold 10 % trichloroacetic acid to a final 3.33 % concentration, then washed 4 times with water. The plates were dried and the cells stained for 30 minutes with 0.057 % (wt/vol) Sulforhodamine B (SRB) in 1% acetic acid, then washed 4 times with 1% acetic acid and dried. The dye was resuspended by a 1 h 30 incubation in 200 μL of 10 mM Tris-base and the absorbance at 490 nm measured with a plate reader (μquant, Bio-tek). For normalization, the background value was subtracted to each value, the duplicates averaged and the untreated condition set to 100% cell viability. CC50 were computed from at least three independent experiments using the GraphPad Prism software using a non-linear regression to a four-parameter logistic curve (log[inhibitor] vs response; variable slope).

**Cytotoxicity evaluation on Vero cell line**

Cytotoxicity evaluation of the tested molecules by MTT assay was performed on the Vero cell line. Briefly, cells ($5.10^4$ cells/mL) in 100 μL of complete medium [MEM supplemented with 10% fetal calf serum (FCS), 2 mM L-glutamine and antibiotics (100U/mL penicillin and 100 μg/mL streptomycin)] were seeded into each well of 96-well plates and incubated at 37 °C in a humidified 5% $CO_2$ with 95% air atmosphere. After a 24 h incubation, 100 μL of medium with various product concentrations and appropriate controls were added and the plates were incubated for 72 h at 37 °C. Each plate-well was then microscope-examined for detecting possible precipitate formation before the medium was aspirated from the wells. 100 μL of MTT solution (0.5 mg/mL in complete MEM) were then added to each well. Cells were incubated for 1 h at 37 °C and the MTT solution was then removed, and DMSO (100 μL/well) was added to dissolve the resulting formazan crystals. Plates were shaken vigorously (300 rpm) for 5 min. The absorbance was measured at 570 nm with a microplate spectrophotometer (BIOTEK Eon). DMSO was used as blank and SDS as positive control. $CC_{50}$ were calculated by non-linear regression analysis processed on dose-response curves, using TableCurve 2D V5 software.

Experiment 1: A series of racemic α-monohydroxylated ($R_3$, $R_4$, $R_5$ and $R_6$ are Hydrogen, X is methyl, n=9) compounds with formula (I-1) were tested and the results are shown in table 1. The $R_2$ group in each molecule varies from saturated groups to unsaturated groups. The comparative example A is the basic model compound in which $R_2$ is hydrogen.

Table 1. Evaluation of biological activity of α-monohydroxylated compounds with formula (I-1) and comparative compound.

| No. of compound | Structure | Anti-*Mtb* MIC (μM) | Anti-HCT116 $CC_{50}$ (μM) | Anti-VERO $CC_{50}$ (μM) | SI ($CC_{50}$/MIC) |
|---|---|---|---|---|---|
| Comparative example A | 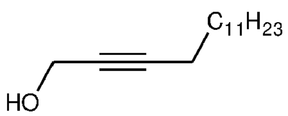 | 5 | 120 | 187.3 | 24 (HCT116)<br>37 (VERO) |
| 18 | 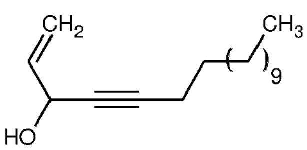 | 2.5 | 0.2 | 1.0 | < 0.1 (HCT116)<br>0.4 (VERO) |
| 19 | 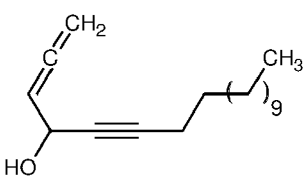 | 2.5 | 2.7 | ND | 1.1 (HCT116) |
| 20 | 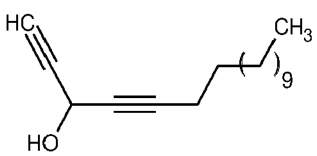 | 1.2 | 0.1 | 0.8 | < 0.1 (HCT116)<br>0.7 (VERO) |

(continued)

| No. of compound | Structure | Anti-*Mtb* MIC (μM) | Anti-HCT116 $CC_{50}$ (μM) | Anti-VERO $CC_{50}$ (μM) | SI ($CC_{50}$/MIC) |
|---|---|---|---|---|---|
| 21 | 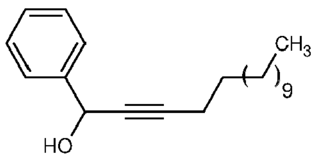 | 1.2 | 50 | 58.7 | 41 (HCT116)<br>42 (VERO) |
| 22 | 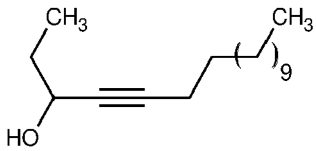 | 2.5 | 12 | > 50 | 5 (HCT116)<br>> 20 (VERO) |
| 23 | 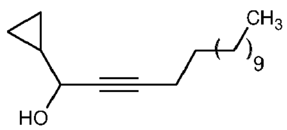 | >10 | 60 | 158.5 | < 6 (HCT116)<br>< 16 (VERO) |
| 24 | 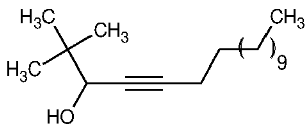 | >10 | 60 | - | < 6 (HCT116) |
| 25 | 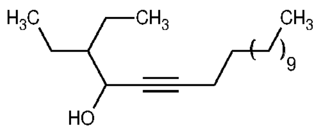 | >10 | 125 | - | < 12 (HCT116) |
| 26 | 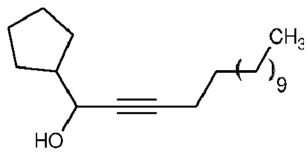 | >10 | 100 | - | < 10 (HCT116) |
| 27 | 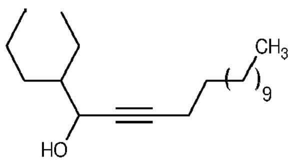 | 10 | 60 | - | 6 (HCT116) |

In view of the Table 1:
In comparison to the comparative example A, introduction of an unsaturated $R_2$ group slightly enhanced the anti-*Mtb* potency while it dramatically favored the anti-HCT116 activity (compounds 18 to 20) at the exception of the compound 21, whose bulkier phenyl head group leads to higher SI because of a relatively low cytotoxicity against HCT116.

In comparison to the compounds with unsaturated $R_2$ groups, compounds with saturated $R_2$ groups were found to be less cytotoxic towards *Mtb* and much less cytotoxic towards HCT116, indicating a better selectivity. In particular, compound with a smaller $R_2$ group, the ethyl compound 22, presents a low anti-*Mtb* MIC inferior to 10 μM and higher SI.

To sum up, compared to the comparative example A, introduction of saturated or unsaturated groups can enhance the cytotoxicity both against *Mtb* and HCT116 cells. The compounds with an unsaturated group exhibit a higher cytotoxicity against *Mtb* and HCT cells than those with a saturated group. On the other hand, the favorable influence of $R_2$ groups on the anti-*Mtb* activity is strongly size-dependent.

Experiment 2: A series of racemic α, α- or α, β- dihydroxylated (one of $R_3$, $R_4$, $R_5$ or $R_6$ is hydroxyl, X is methyl, n=9) compounds with formula (I-1) were tested and the results are shown in Table 2.

Table 2. Evaluation of biological activity of α, α- or α, β- dihydroxylated compounds with formula (I-1) and comparative compound.

| No. of compound | Structure | Anti-*Mtb* MIC (μM) | Anti-HCT116 $CC_{50}$ (μM) | Anti-VERO $CC_{50}$ (μM) | SI ($CC_{50}$/MIC) |
|---|---|---|---|---|---|
| Comparative example A | 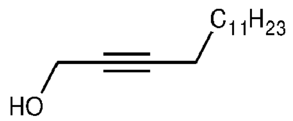 | 5 | 120 | 187.3 | 24 (HCT116)<br>37 (VERO) |
| 1 | 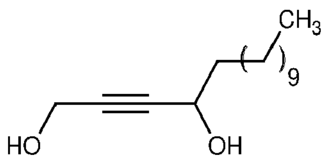 | 1.25 | 60 | 89.0 | 48 (HCT116)<br>71 (VERO) |
| 2 | 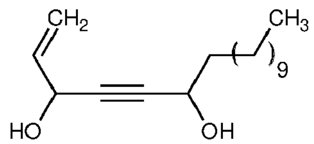 | 1.25 | 2 | 7.6 | 1.6 (HCT116)<br>6 (VERO) |
| 3 | 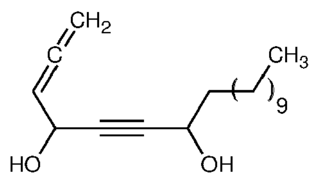 | 2.5 | 9 | 8 | 3.6 (HCT116)<br>3.2 (VERO) |
| 4 | 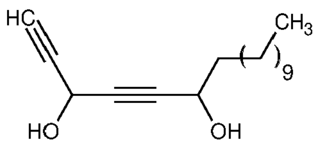 | 0.3 | 4 | 2.5 | 13 (HCT116)<br>8 (VERO) |
| 5 | 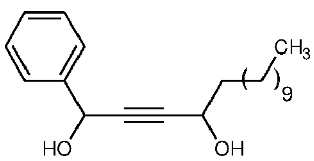 | 1.25 | 50 | 46.5 | 40 (HCT116)<br>37 (VERO) |
| 6 | 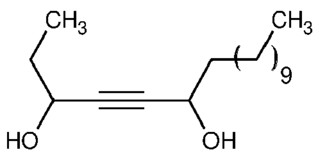 | 2.5 | 40 | 43.2 | 16 (HCT116)<br>17 (VERO) |
| 7 | 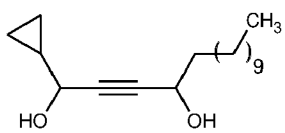 | 0.3 | 50 | 43.9 | 167 (HCT116)<br>146 (VERO) |
| 8 | 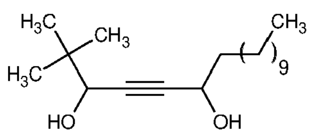 | 5 | 20 | ND | 4 (HCT116) |
| 9 | 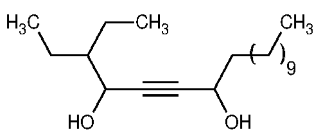 | 5 | 20 | ND | 4 (HCT116) |

(continued)

| No. of compound | Structure | Anti-*Mtb* MIC (μM) | Anti-HCT116 $CC_{50}$ (μM) | Anti-VERO $CC_{50}$ (μM) | SI ($CC_{50}$/MIC) |
|---|---|---|---|---|---|
| 10 | 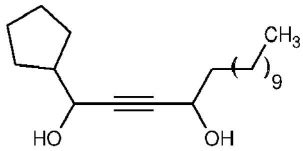 | 5 | 20 | ND | 4 (HCT116) |
| 11 | 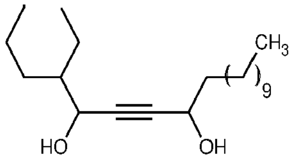 | 5 | 50 | ND | 4 (HCT116) |
| 12 | 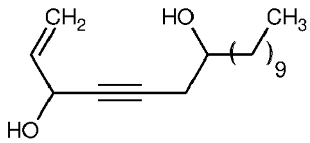 | 1.25 | 2 | 12.5 | 1.6 (HCT116)<br>10 (VERO) |
| 13 | 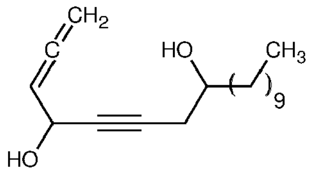 | 0.6 | 12 | ND | 20 (HCT116) |
| 14 | 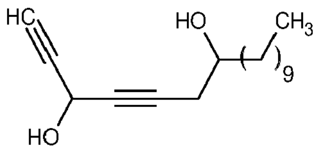 | 0.6 | 2 | 173.9 | 3 (HCT116)<br>290 (VERO) |
| 15 | 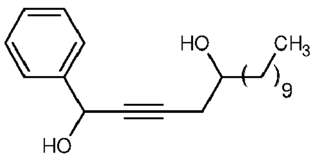 | 2.5 | 41 | 75 | 16 (HCT116)<br>30 (VERO) |
| 16 | 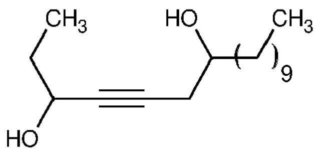 | 5 | 51 | 75 | 10 (HCT116)<br>15 (VERO) |
| 17 | 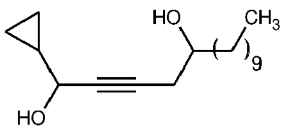 | 0.6 | 40 | 44.5 | 67 (HCT116)<br>74 (VERO) |

In view of the Table 2:
In comparison to the compound A, the introduction of the second group of hydroxyl leads to a significant increase in anti-*Mtb* activity and higher selectivity, as illustrated for the compound 1.

In comparison to the compound 1, the introduction of an unsaturated or saturated $R_2$ group can slightly increase the anti-*Mtb* activity.

For compounds 6 to 11, in comparison to their corresponding α-monohydroxylated compounds 22 to 27 in the Table 1, the presence of the second hydroxyl group next to the triple bond substantially improves the anti-*Mtb* activity, and leads to a higher selectivity.

Among compounds 6 to 11, compounds 6 and 7 with relatively smaller head group $R_2$ (ethyl, cyclopropyl) present

higher SI values of 16 and 167, respectively, in comparison with those of 8 to 11 with bulkier head groups (t-butyl, 1-ethylpropyl, cyclopentyl, cyclohexyl) with SI=4.

Compounds 12 to 17, in which the second hydroxyl group was shifted to the homopropargylic position, in comparison to their corresponding dihydroxylated analogues with hydroxyl groups in both the propargylic positions (6 to 11), give equivalent or slightly reduced SI and anti-*Mtb* activity.

To sum up, the introduction of the second hydroxyl group at the propargylic or homopropargylic position of compounds with formula (I-1) can significantly increase the anti-*Mtb* activity and the selectivity. Among the compounds in Table 2, compounds 1, 5, 6, 7, 13, 14, 15, 16, 17 are considered as the most promising molecules for treatment against tuberculosis, exhibiting a low anti-*Mtb* MIC value and high SI values.

Experiment 3: A series of enantiomers separated from compounds 1 or 6 with formula (I-1) were tested and the results are shown in Table 2.

Table 3. Evaluation of anti-*Mtb* activity of enantiomers separated from compounds 1 or 6 with formula (I-1).

| No. of compound | Structure | Anti-*Mtb* MIC (μM) |
|---|---|---|
| (S)-1 | 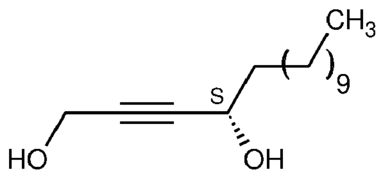 | 1.25 |
| (R)-1 | 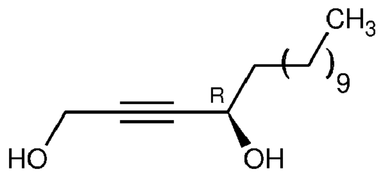 | 1.25 |
| (S,S)-6 | 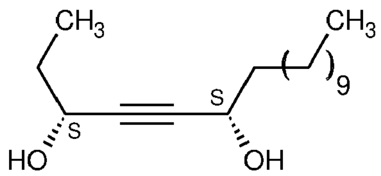 | 2.5 |
| (R,R)-6 | 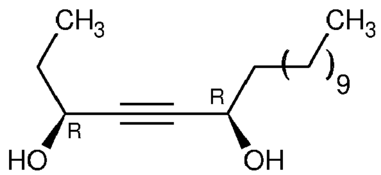 | 2.5 |
| (S,R)-6 | 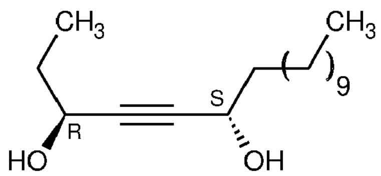 | 2.5 |
| (R,S)-6 | 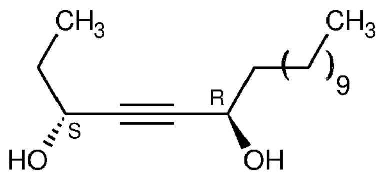 | 2.5 |

In view of the Table 3:
The anti-*Mtb* activity of compounds with asymmetrical carbons does not depend on the configuration of the molecules. Enantiomers of the same molecules have equivalent anti-*Mtb* activity.

Table 4. Antibacterial activity evaluated with *Mtb* and other bacterial species.

| **No. of compound** | **Structure** | **Antibacterial activity MIC (μM)** | | | | | |
|---|---|---|---|---|---|---|---|
| | | *E. coli* | *C. glutamicum* | *M. tuberculosis* | *M. smegmatis* | *M. kansasii* | *M. abscessus* |
| (S)-20 | 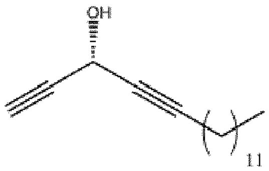 | >10 | 10 | 1.2 | 2.5 | 1.2 | |
| (S)-19 | 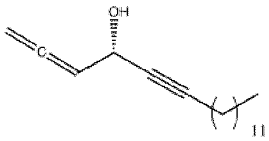 | >10 | >10 | 2.5 | 10 | 2.5 | |
| (R)-19 | 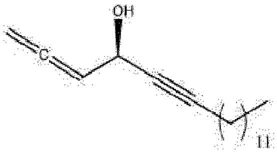 | >10 | >10 | 2.5 | 10 | 5 | |
| 28 | 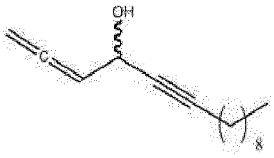 | >10 | >10 | 5 | >10 | 10 | |
| 22 | 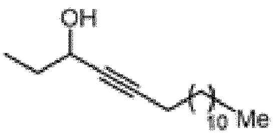 | | | 2.5 | | | >20 |
| 6 | 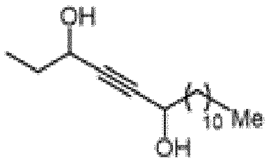 | | | 2.5 | | | 10 |
| Comparative example A | 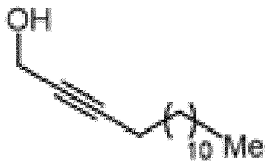 | | | 5 | | | 20 |
| 1 | 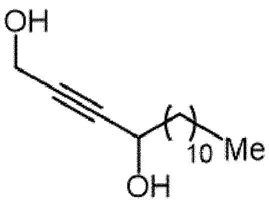 | | | 1.25 | | | 2.5 |

In view of the Table 4:
*Escherichia coli* (*E.* coli) and *Corynebacterium glutamicum* (*C. glutamicum*) are non-mycobacteria. *M. smegmatis, M. kansasii,* and *M. abscessus* are non-tuberculous mycobacteria.

Compounds listed in the Table 4 exhibiting anti-*Mtb* activity also show antibacterial effect towards other types of

mycobacteria while are not or less active towards non-mycobacteria such as *E. coli* and *C. glutamicum.*

Compared to compound 22, the compound 6, with a second hydroxyl group at the propargylic position, shows higher activity against *M. abscesses.*

Compared to comparative example A, the compound 1, with a second hydroxyl group at the propargylic position, shows higher activity towards both *Mtb* and *M. abscesses.*

In conclusion, compounds of the present invention show antibacterial activity not only towards *Mtb,* but also towards other kind of mycobacteria, while they are less active against other non-mycobacterial species. In addition, antibacterial activity against other kind of mycobacteria can be enhanced via introduction of the second hydroxyl group at the propargylic position of the compounds.

## Claims

**1.** A compound of Formula (I)

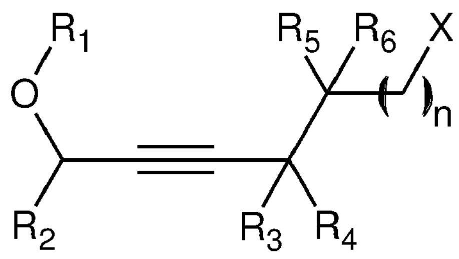

(I)

wherein:

- n is an integer comprised between 4 and 15;
- $R_1$ is Hydrogen, or a -C(=O)-R' or an alcohol protecting group;
- $R_2$ is chosen from the group consisting of: Hydrogen, Alkyl, Cycloalkyl, Heterocycle, Alkenyl, Allenyl, Alkynyl, Aryl, and Heteroaryl;
- $R_3$, $R_4$, $R_5$ and $R_6$ identical or different are independently chosen from the group consisting of Hydrogen, Halogen, Alkyl, Alkenyl, Alkynyl, AlkylAryl, $OR_a$, $COOR_b$, $NR_cR_d$, wherein at least one of $R_3$, $R_4$, $R_5$ and $R_6$ is different from H;
- Alternatively, groups $R_3$ and $R_4$ or groups $R_5$ and $R_6$ can form double carbon-oxygen bond (C=O);
- X is selected from the group consisting of $CH_3$, $CH_2F$, $CHF_2$, $CHFCH_2F$, $CF_3$, COOH, $CH_2OH$, alkoxy, CCSi(i-Pr)$_3$, alkynyl, $NH_2$, -O-P(=O)($O_2$)M and -O-S(=O)$_2$-OM;

wherein:

- M is one or more mono- or divalent metal ions chosen from $Li^+$, $Na^+$, $Mg^{2+}$, $K^+$ and $Ca^{2+}$;
- $R_a$, $R_b$, $R_c$, $R_d$, identical or different, are independently chosen from H, Alkyl and - C(=O)-R' groups;
- R' is chosen from Hydrogen, Alkyl, Halogen, $OR_e$, $NR_fR_g$ Cycloalkyl, and Aryl, wherein $R_e$, $R_f$, $R_g$ identical or different, are independently chosen from H and Alkyl;

wherein each of Alkyl, Alkenyl, Allenyl, Alkynyl, Cycloalkyl, Heterocycle, Aryl, Heteroaryl may be optionally substituted by one or more group chosen from the group consisting of: halogen, $OR_a$, $COOR_b$, $NR_cR_d$, CN, $NO_2$, $CF_3$; wherein:

- Alkyl refers to a linear or branched alkyl group;
- Alcohol protecting group refers to Aryl, Heterocycles, alkyl ether, silyl, or glycosyl;
- Cycloalkyl refers to a mono, bi or tricyclic carbocyclic ring;
- Heterocycle refers to a mono, bi or tricyclic saturated or unsaturated ring comprising one or more Heteroatoms chosen from N, O and S;
- Alkenyl refers to a linear or branched alkenyl group comprising one or more double bonds;
- Allenyl group refers to a -C($R_a$)=C=C$R_bR_c$ group;
- Alkynyl refers to a linear or branched alkynyl, comprising 1 or more triple bonds;
- Aryl refers to a mono, or bicyclic aromatic ring;

- Heteroaryl refers to a mono or bicyclic aromatic ring comprising one or more Heteroatoms chosen from N, O and S;
- Halogen is chosen from F, Cl, Br and I;
as well as the stereoisomers thereof,
or a pharmaceutically acceptable salt or ester thereof.

**2.** A compound according to claim 1, wherein $R_1$ is Hydrogen.

**3.** A compound according to claim 1 or 2, wherein $R_2$ is selected from Alkyl, Alkynyl, Cycloalkyl, Aryl, and Heteroaryl.

**4.** A compound according to any one of claims 1, 2 or 3, wherein $R_3$, $R_4$, $R_5$ and $R_6$ identical or different are each independently chosen from the group consisting of Hydrogen and Hydroxyl, wherein at least one of $R_3$, $R_4$, $R_5$ and $R_6$ is Hydroxyl.

**5.** A compound according to any one of the preceding claims, wherein X is selected from $-CH_3$, $-COOH$, $-CH_2OH$, $-NH_2$, Alkynyl, $-CH_2F$, $-CHF_2$, and $-CF_3$..

**6.** A compound according to any one of the preceding claims, wherein n is 9 or 10.

**7.** A compound according to any one of the preceding claims, selected from the group consisting of:

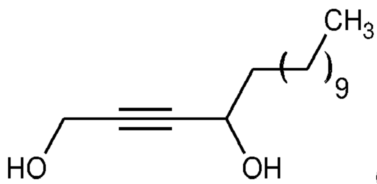 (1)

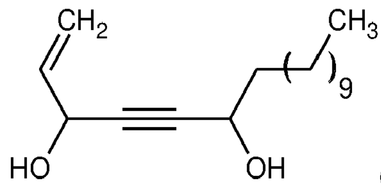 (2)

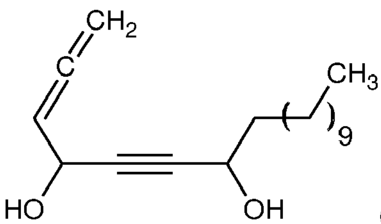 (3)

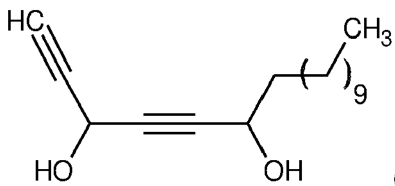 (4)

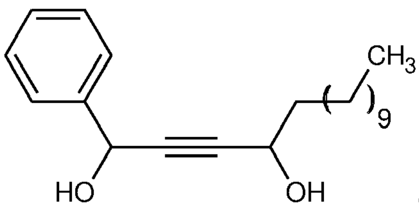 (5)

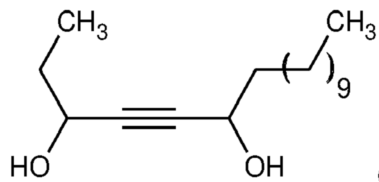 (6)

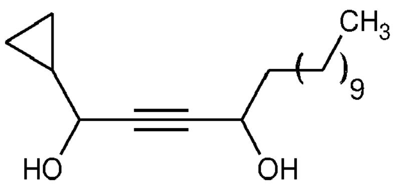 (7)

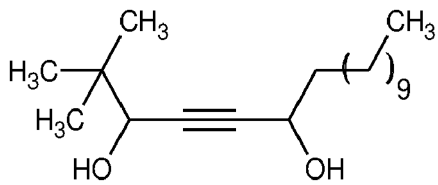 (8)

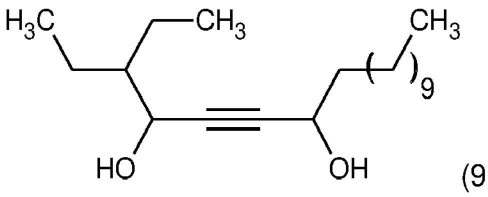 (9) 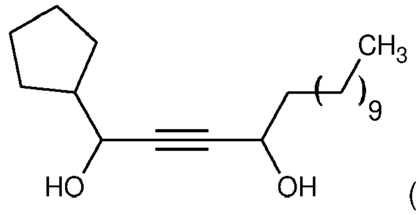 (10)

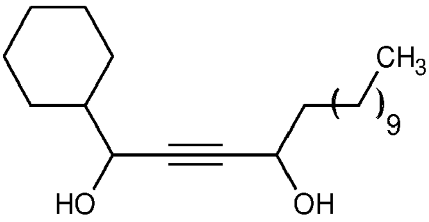 (11) 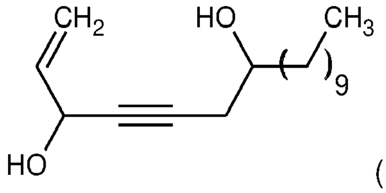 (12)

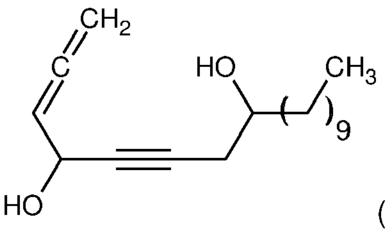 (13) 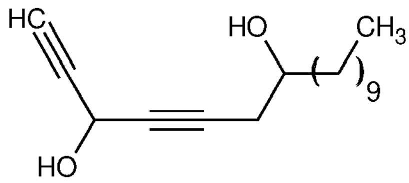 (14)

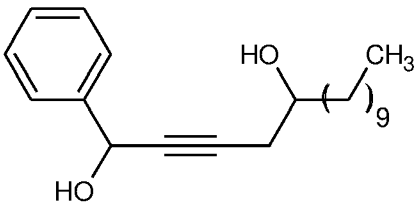 (15) 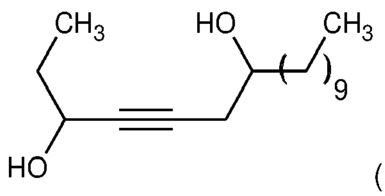 (16)

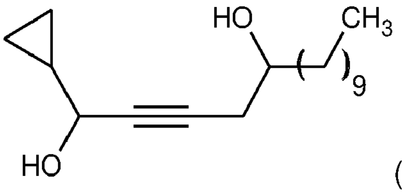 (17)

as well as the stereoisomers thereof,
or a pharmaceutically acceptable salt or ester thereof.

**8.** A compound according to any one of the preceding claims, selected from the group consisting of:

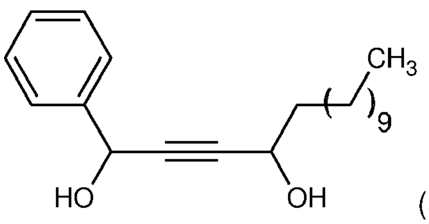 (5) 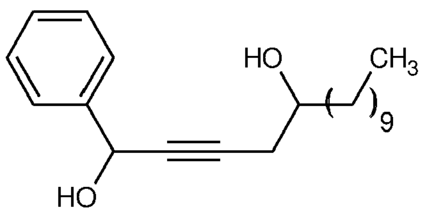 (15)

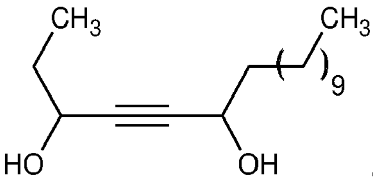
(6)

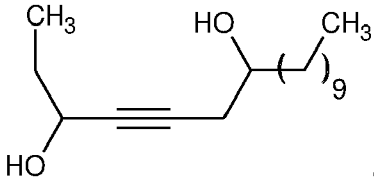
(16)

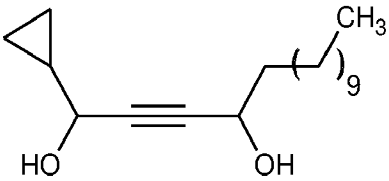
(7)

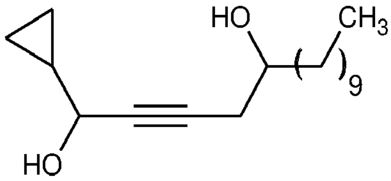
(17)

as well as the stereoisomers thereof,
or a pharmaceutically acceptable salt or ester thereof.

**9.** A compound according to any one of the preceding claims of formula (II):

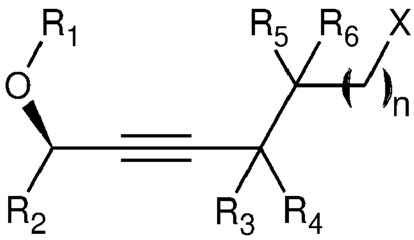
(II)

or a pharmaceutically acceptable salt or ester thereof.

**10.** A process of preparation of a compound with formula (I) according to anyone of the preceding claims, said process comprising a step of condensation reaction between a metal acetylide (D):

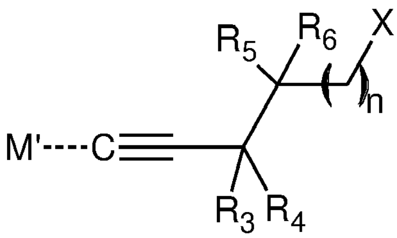
(D)

wherein M' represents an alkali metal based ion, in particular a Lithium ion or a Magnesium bromide ion, and the dashed bond between the M' and the carbon atom represents a covalent bond and/or an ionic bond;
and a precursor (C):

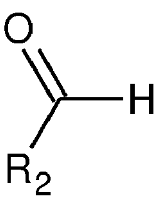
(C)

leading to compound (A) of formula:

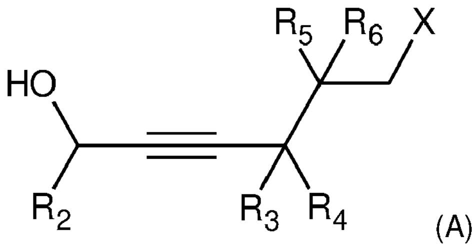

(A)

optionally followed by the reaction between the compound (A) and a precursor (B):

$R_1$-Z (B)

wherein Z refers to Halogen, mesylate, or tosylate group, leading to compound of formula (I):

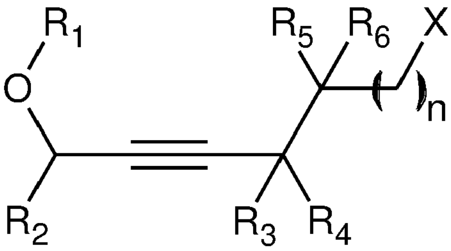

(I)

wherein $R_1$, $R_2$, $R_3$, $R_4$, $R_5$, $R_6$ and X are defined as in any one of claims 1 to 9.

**11.** A pharmaceutical composition, comprising at least one compound of formula (I) according to any one of claims 1 to 9, together with at least one pharmaceutically acceptable carrier.

**12.** A compound according to any one of claims 1 to 9, for use in the treatment of infections caused by bacteria, parasites or fungus.

**13.** A compound with formula (I"), for use in the treatment of infections caused by bacteria, parasites or fungus:

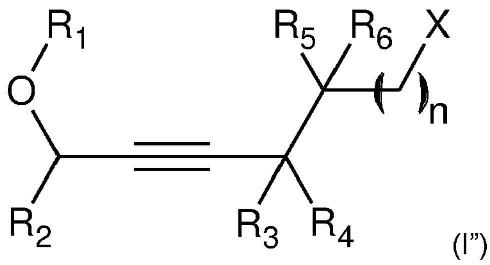

(I"),

wherein:

- n is an integer comprised between 4 and 15;
- $R_1$ is Hydrogen, or a -C(=O)-R' or an alcohol protecting group;
- $R_2$ is chosen from the group consisting of: Hydrogen, Alkyl, Cycloalkyl, Heterocycle, Alkenyl, Allenyl, Alkynyl, Aryl, and Heteroaryl;
- $R_3$, $R_4$, $R_5$ and $R_6$ identical or different are independently chosen from the group consisting of Hydrogen, Halogen, Alkyl, Alkenyl, Alkynyl, AlkylAryl, $OR_a$, $COOR_b$, $NR_cR_d$;
- Alternatively, groups $R_3$ and $R_4$ or groups $R_5$ and $R_6$ can form double carbon-oxygen bond (C=O);
- X is selected from the group consisting of $CH_3$, $CH_2F$, $CHF_2$, $CHFCH_2F$, $CF_3$, COOH, $CH_2OH$, alkoxy, CCSi(i-Pr)$_3$, alkynyl, $NH_2$, -O-P(=O)($O_2$)M and -O-S(=O)$_2$-OM;

wherein M, $R_a$, $R_b$, $R_c$, $R_d$ and R' are defined according to the claim 1;

as well as the stereoisomers thereof,
or a pharmaceutically acceptable salt or ester thereof.

**14.** The compound for use according to claims 12 or 13 wherein said infections are caused by *Mycobacterium.*

**15.** The compound for use according to according to claims 12, 13 or 14 wherein said infections are caused by *Mycobacterium tuberculosis* and/or *Mycobacterium bovis* and/or *Mycobacterium abscessus* and/or *Mycobacterium kansasii* and/or *Mycobacterium avium and/or Mycobacterium smegmatis.*

**16.** The compound for use according to any one of claims 12 to 15 wherein said infections are caused by *Mycobacterium tuberculosis.*

Europäisches Patentamt
European Patent Office
Office européen des brevets

# EUROPEAN SEARCH REPORT

Application Number
EP 24 30 5335

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LI CAN ET AL: "A Pd-catalyzed highly selective three-component protocol for trisubstituted allenes", CHEMICAL SCIENCE, vol. 14, no. 28, 19 July 2023 (2023-07-19), pages 7709-7715, XP093239450, United Kingdom ISSN: 2041-6520, DOI: 10.1039/D3SC01849K * Supplementary information, reactions (11) and (13); page S11 - page S12; compound 1n * | 1,2,4,5 | INV. C07C33/044 C07C33/048 C07C33/05 C07C33/12 C07C33/14 C07C33/30 |
| X | KAWASE TOKUZO ET AL: "Novel Synthesis of Anionic Gemini Surfactants from 1, 4-Diol as a Key Block Material", JOURNAL OF OLEO SCIENCE, vol. 64, no. 9, 2015, pages 971-986, XP093239469, JP ISSN: 1345-8957, DOI: 10.5650/jos.ess15109 * page 976, right-hand column, last paragraph - page 977; compounds 8-9 * | 1-6,10 | |
| X | WO 2019/010414 A1 (US HEALTH [US]) 10 January 2019 (2019-01-10) * page 34; compound 275 * | 1-5,11 | TECHNICAL FIELDS SEARCHED (IPC) C07C |

-/--

The present search report has been drawn up for all claims

1

| Place of search | Date of completion of the search | Examiner |
|---|---|---|
| The Hague | 20 January 2025 | Kövecs, Monika |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 4

European Patent Office
Europäisches Patentamt
Office européen des brevets

# EUROPEAN SEARCH REPORT

Application Number
EP 24 30 5335

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YADAV J S ET AL: "The first total synthesis of the 6-hydroxy-4E-sphingenines", TETRAHEDRON LETTERS, ELSEVIER, AMSTERDAM, NL, vol. 44, no. 14, 31 March 2003 (2003-03-31), pages 2983-2985, XP004414414, ISSN: 0040-4039, DOI: 10.1016/S0040-4039(03)00390-3 * Scheme 3; compounds 12a-b * ----- | 1-6 | |
| X | RAO WEIDONG ET AL: "Ligand-Controlled Product Selectivity in Gold-Catalyzed Double Cycloisomerization of 1,11-Dien-3,9-Diyne Benzoates", JOURNAL OF THE AMERICAN CHEMICAL SOCIETY, vol. 137, no. 19, 20 May 2015 (2015-05-20), pages 6350-6355, XP093239544, ISSN: 0002-7863, DOI: 10.1021/jacs.5b02377 * Supporting Information; pages S9, S16 - page S18; compounds 1a, 1n, 1o, 1p, 1q * * Supporting Information, method A; page S4; compound S3 * ----- | 1-3,5,6 | TECHNICAL FIELDS SEARCHED (IPC) |
| X | DATABASE REGISTRY [Online] Chemical Abstracts Service, Columbus, OH, US; 14 June 2021 (2021-06-14), XP093239677, Database accession no. 2646323-83-1 * abstract * ----- -/-- | 1,2,5 | |

The present search report has been drawn up for all claims

1

| Place of search | Date of completion of the search | Examiner |
|---|---|---|
| The Hague | 20 January 2025 | Kövecs, Monika |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 4

Europäisches Patentamt
European Patent Office
Office européen des brevets

# EUROPEAN SEARCH REPORT

Application Number
EP 24 30 5335

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE REGISTRY [Online] Chemical Abstracts Service, Columbus, OH, US; 14 January 2021 (2021-01-14), XP093239673, Database accession no. 2570267-15-9 * abstract * | 1-3,5 | |
| X | DATABASE REGISTRY [Online] Chemical Abstracts Service, Columbus, OH, US; 10 March 2021 (2021-03-10), XP093239674, Database accession no. 2607427-68-7 * abstract * | 1-3,5 | |
| X | DATABASE REGISTRY [Online] Chemical Abstracts Service, Columbus, OH, US; 5 May 2021 (2021-05-05), XP093239670, Database accession no. 2603949-83-1 * abstract * | 1-3,5,9 | TECHNICAL FIELDS SEARCHED (IPC) |
| X | FEIXAS J. ET AL: "Synthesis of (z)-10,10-difluoro-13-hexadecen-11-ynyl acetate, new difluoro analogue of the sex pheromone of the processionary moth", BIOORGANIC & MEDICINAL CHEMISTRY LETTERS, vol. 2, no. 5, 1999, pages 467-470, XP093239754, Amsterdam NL ISSN: 0960-894X * Scheme 1; compound 12 * | 1,2 | |

-/--

The present search report has been drawn up for all claims

1

| Place of search | Date of completion of the search | Examiner |
|---|---|---|
| The Hague | 20 January 2025 | Kövecs, Monika |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 24 30 5335

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FRÉDÉRIC JUSTAUD ET AL: "Development of the Meyer-Schuster Rearrangement on Propargylic Alcohols with Fluorinated Chains", EUROPEAN JOURNAL OF ORGANIC CHEMISTRY, WILEY-VCH, DE, vol. 26, no. 47, 22 November 2023 (2023-11-22), page n/a, XP072552126, ISSN: 1434-193X, DOI: 10.1002/EJOC.202300974 * Scheme 2; compounds 1-4 * * Supporting Information; page S4 * ----- | 1-3,5,10 | |
| X | US 2 783 258 A (CELMER WALTER D) 26 February 1957 (1957-02-26) | 13-16 | |
| A | * example 1; compound I * * column 6, line 65 - line 71 * ----- | 7,8,12 | |
| | | | TECHNICAL FIELDS SEARCHED (IPC) |

The present search report has been drawn up for all claims

1

| Place of search | Date of completion of the search | Examiner |
|---|---|---|
| The Hague | 20 January 2025 | Kövecs, Monika |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 30 5335

This annex lists the patent family members relating to the patent documents cited in the above-mentioned European search report. The members are as contained in the European Patent Office EDP file on
The European Patent Office is in no way liable for these particulars which are merely given for the purpose of information.

20-01-2025

| Patent document cited in search report | Publication date | Patent family member(s) | Publication date |
|---|---|---|---|
| WO 2019010414 A1 | 10-01-2019 | AU 2018297192 A1 | 19-12-2019 |
| | | CA 3064132 A1 | 10-01-2019 |
| | | CN 110869354 A | 06-03-2020 |
| | | EP 3649115 A1 | 13-05-2020 |
| | | JP 7234151 B2 | 07-03-2023 |
| | | JP 2020526505 A | 31-08-2020 |
| | | KR 20200027545 A | 12-03-2020 |
| | | US 2021139440 A1 | 13-05-2021 |
| | | US 2022274940 A1 | 01-09-2022 |
| | | WO 2019010414 A1 | 10-01-2019 |
| US 2783258 A | 26-02-1957 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **STOVER et al.** *Nature*, June 2000, vol. 405, 962-966
- **MATSUMOTO et al.** *PLoS Med.*, November 2006, vol. 3 (11), e466
- **ANDRIES et al.** *Science*, January 2005, vol. 307 (5707), 223-227
- **PALOMINO et al.** *Curr. Med. Chem.*, May 2009, vol. 16 (15), 1898-1904
- **ROGOZA et al.** *Chem. Sustain. Development*, 2010, vol. 10, 343-375
- **KOCH et al.** *Planta Med.*, 2010, vol. 75 (15), 1678-1682
- **MORBIDONI et al.** *Chemistry & Biology*, March 2006, vol. 13 (3), 297-307
- **LI et al.** *J. Ethnopharmacol.*, March 2012, vol. 140 (1), 141-144
- **E! ARFAOUI et al.** *ChemMedChem.*, September 2013, vol. 8, 1779-1786
- **LISTUNOV et al.** *Synthesis*, 2018, vol. 50 (16), 3114-3130
- **DEMANGE**. *eLife*, May 2022, vol. 1, e73913
- **BOSSUAT et al.** *J. Med. Chem.*, October 2023, vol. 66 (20), 13918-13945
- **MORBIDONI et al.** Dual inhibition of mycobacterial fatty acid biosynthesis and degradation by 2-alkynoic acids. *Chemistry & Biology*, 2006, vol. 13 (3), 297-307
- **DENG et al.** Anti-TB Polyynes from Roots of Angelica sinensis. *Phytotherapy Research*, 2008, vol. 22, 878-882
- **EL ARFAOUI, D et al.** *ChemMedChem*, 2013, vol. 8, 1779-1786
- **LISTUNOV, D et al.** *ChemMedChem*, 2018, vol. 13, 1711-1722
- **BOURKHIS, M et al.** *ChemMedChem*, 2018, vol. 13, 1124-1130
- **LISTUNOV, D et al.** *Tetrahedron*, 2015, vol. 71, 7920-7930